(19) Europäisches Patentamt
European Patent Office
Office européen des brevets

(11) EP 3 569 229 A1

# (12) EUROPEAN PATENT APPLICATION
published in accordance with Art. 153(4) EPC

(43) Date of publication:
**20.11.2019 Bulletin 2019/47**

(21) Application number: **18739182.6**

(22) Date of filing: **11.01.2018**

(51) Int Cl.:
***A61K 31/18*** *(2006.01)* ***A61P 25/00*** *(2006.01)*
***A61P 25/24*** *(2006.01)* ***A61P 43/00*** *(2006.01)*

(86) International application number:
**PCT/JP2018/000531**

(87) International publication number:
**WO 2018/131662 (19.07.2018 Gazette 2018/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **11.01.2017 JP 2017002962**

(71) Applicant: **Public University Corporation Yokohama City University**
**Yokohama-shi, Kanagawa 236-0027 (JP)**

(72) Inventors:
- **TAKAHASHI, Takuya**
  **Yokohama-shi**
  **Kanagawa 236-0004 (JP)**
- **MIYAZAKI, Tomoyuki**
  **Yokohama-shi**
  **Kanagawa 236-0004 (JP)**
- **NAKAJIMA, Waki**
  **Yokohama-shi**
  **Kanagawa 236-0004 (JP)**

(74) Representative: **Prüfer & Partner mbB**
**Patentanwälte · Rechtsanwälte**
**Sohnckestraße 12**
**81479 München (DE)**

## (54) PROPHYLACTIC AND/OR THERAPEUTIC AGENT FOR DISEASES ASSOCIATED WITH AMPA RECEPTORS

(57) This prophylactic and/or therapeutic agent for diseases associated with AMPA receptors contains a compound represented by formula (I), or a pharmaceutically acceptable salt or solvate thereof. (In the formula, A and Z independently represent CO, SO or $SO_2$; X and Y are independently S or O; $R^1$-$R^4$ are independently hydrogen, an alkyl group, an alkenyl group, an alkynyl group or a halo group; $R^5$ is, independently for each occurrence, an alkyl group, an alkenyl group, an alkynyl group or a halo group; and n is an integer of 0-4).

EP 3 569 229 A1

**Description**

TECHNICAL FIELD

The present invention relates to a prophylactic and/or therapeutic agent for diseases associated with AMPA receptors, and more particularly relates to a therapeutic agent for a disease in which the amount of AMPA receptor is lowered.

BACKGROUND ART

AMPA receptors are synapse functional molecules, and play an important role in mental and nervous activities. The present inventors have clarified that the synaptic delivery of AMPA receptors (glutamate receptors) in vivo is the molecular cell mechanism of the plasticity phenomena of various higher brain functions such as memory learning (Non-Patent Documents 1 to 8). In recent years, mainly in the postmortem brain research field, it has been reported that the AMPA receptors are involved with a large number of mental and neurological diseases such as depression, schizophrenia, drug dependence, autism, epilepsy disease and ALS. For the diseases described above, the preclinical and clinical development of AMPA receptor agonists is actively performed. AMPA receptor-targeted drugs (AMPA receptor agonists) are roughly divided into (1) a receptor antagonist and (2) a receptor function activator. As the former, an AMPA receptor antagonist for epilepsy disease (Fycompa; Eisai Co., Ltd.) was launched in Japan in March 2016. On the other hand, no AMPA receptor function activator has so far been launched. Since the mechanism of occurrence of the diseases described above is heterogeneous, it is considered unlikely that the AMPA receptor agonist is effective for all cases. Hence, it is required to develop a pharmaceutical composition which contains a compound that indicates an AMPA receptor function activation action.

Non-Patent Document 1: Takahashi,Science 2003.
Non-Patent Document 2: Mitsushima,Takahashi,PNAS 2011.
Non-Patent Document 3: Jitsuki,Takahashi,Neuron 2011.
Non-Patent Document 4: Miyazaki,Takahashi,J. Clinical Investigation 2012.
Non-Patent Document 5: Miyazaki,Takahashi,European J. Neurosci.2013.
Non-Patent Document 6: Mitsushima,Takahashi,Nature Communications 2013.
Non-Patent Document 7: Tada,Miyazaki,Takahashi,PNAS 2016.
Non-Patent Document 8: Takemoto,Takahashi,Nat.Biotech.2016

DISCLOSURE OF THE INVENTION

Problems to be Solved by the Invention

An object of the present invention is to provide a prophylactic and/or therapeutic agent for diseases associated with the AMPA receptors. In particular, an object of the present invention is to provide a prophylactic and/or therapeutic agent for a disease in which the amount of AMPA receptor is lowered.

Means for Solving the Problems

The present inventors have conducted intensive studies so as to find that a compound represented by formula (I) below or a pharmaceutically acceptable salt or solvate thereof indicates high brain delivery so as to indicate an AMPA receptor function activation action, and thereby have completed the present invention.

[Chem. 1]

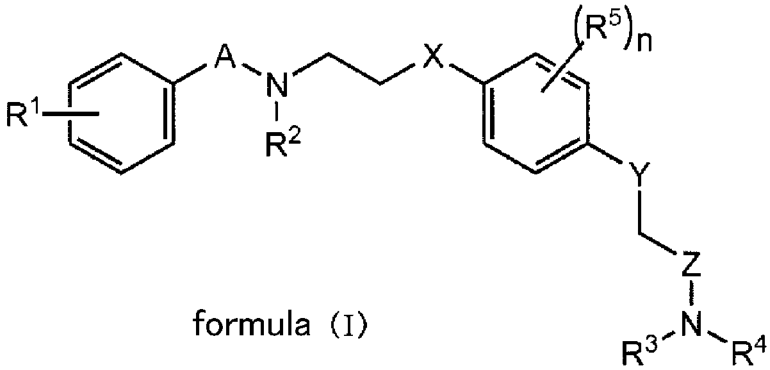

formula (I)

Specifically, the present invention is as follows.

(1) A prophylactic and/or therapeutic agent for a disease associated with AMPA receptors, including a compound of formula (I) or a pharmaceutically acceptable salt or solvate thereof:

[Chem. 2]

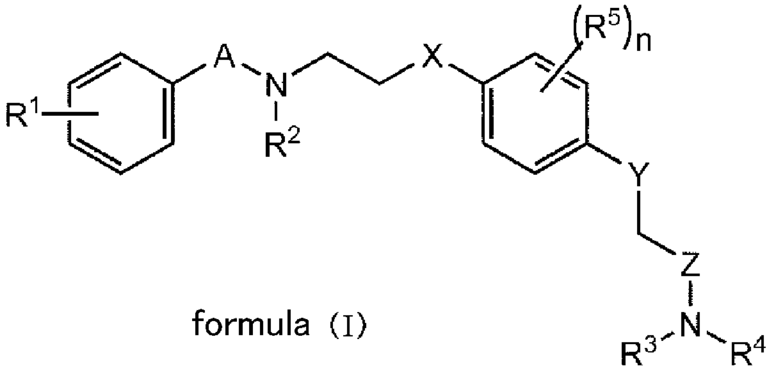

formula (I)

(in the formula,

each of A and Z independently represents CO, SO or $SO_2$;
each of X and Y independently represents S or O;
each of $R^1$ to $R^4$ independently represents hydrogen, alkyl, alkenyl, alkynyl or halo;
each $R^5$ independently represents alkyl, alkenyl, alkynyl or halo;
each $R^5$ independently represents alkyl, alkenyl, alkynyl or halo; and
n represents an integer of 0 to 4).

(2) The prophylactic and/or therapeutic agent described in (1) above, in which not all of $R^1$ to $R^4$ are hydrogen.
(3) The prophylactic and/or therapeutic agent described in (1) or (2) above, in which each of A and Z independently represents CO or $SO_2$.
(4) The prophylactic and/or therapeutic agent described in any one of (1) to (3) above, in which A represents $SO_2$ and Z represents CO.
(5) The prophylactic and/or therapeutic agent described in any one of (1) to (4) above, in which X represents S and Y represents O.
(6) The prophylactic and/or therapeutic agent described in any one of (1) to (5) above, in which $R^2$ represents alkyl,

alkenyl or alkynyl.

(7) The prophylactic and/or therapeutic agent described in (6) above, in which $R^2$ represents alkyl.

(8) The prophylactic and/or therapeutic agent described in any one of (1) to (7) above, in which $R^1$ represents alkyl or halo.

(9) The prophylactic and/or therapeutic agent described in any one of (1) to (8) above, in which one of $R^3$ and $R^4$ represents hydrogen and the other represents alkyl.

(10) The prophylactic and/or therapeutic agent described in any one of (1) to (9) above, in which $R^5$ represents halo.

(11) The prophylactic and/or therapeutic agent described in (10) above, in which the halo is fluoro.

(12) The prophylactic and/or therapeutic agent described in any one of (1) to (11) above, in which n represents 2.

(13) The prophylactic and/or therapeutic agent described in (1) above, in which the prophylactic and/or therapeutic agent is selected from the group consisting of compounds below:

[Chem. 3]

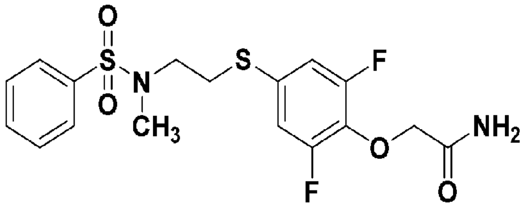
;
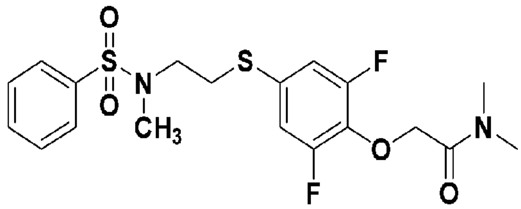

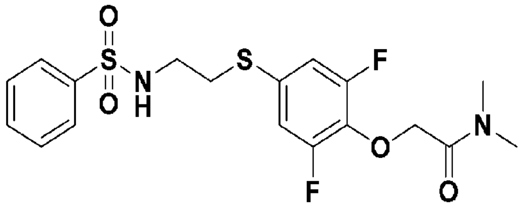
;
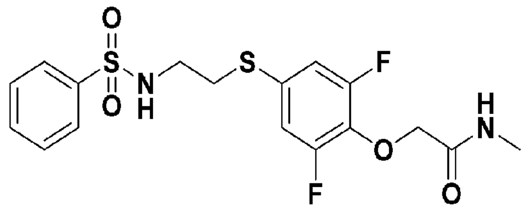

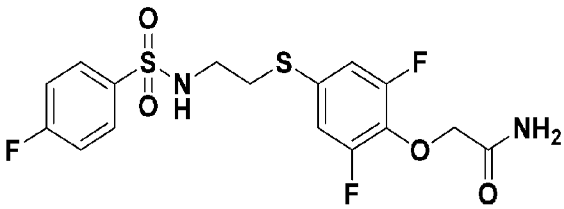
;
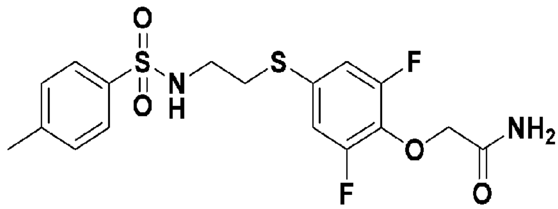

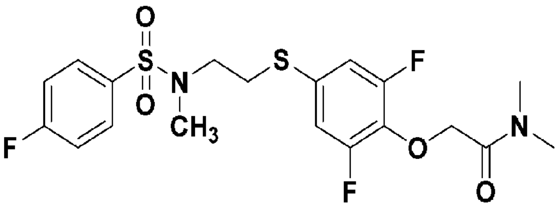
;
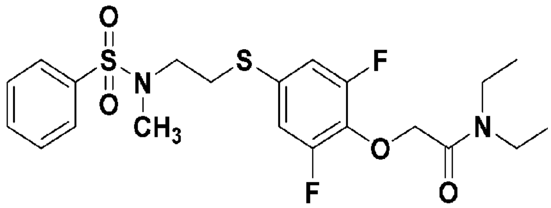

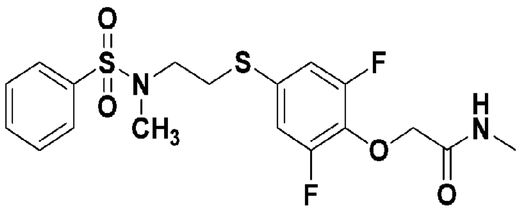

(14) The prophylactic and/or therapeutic agent described in (1) to (13) above, in which the prophylactic and/or therapeutic agent is an AMPA receptor function activator.

(15) The prophylactic and/or therapeutic agent described in any one of (1) to above, in which the disease associated with the AMPA receptors is a mental disease or a neurological disease.

(16) The prophylactic and/or therapeutic agent described in (15) above, in which the mental disease is depression.

Effects of the Invention

According to the present invention, it is possible to provide a prophylactic and/or therapeutic agent for diseases associated with AMPA receptors which contains the compound of formula (I) or a pharmaceutically acceptable salt or solvate thereof.

BRIEF DESCRIPTION OF THE DRAWINGS

Fig. 1 is a graph showing the result of an AMPA receptor binding test (in vitro autoradigraphy method) in vitro on K-2;
Fig. 2 is a graph showing the result of an AMPA receptor binding test (electrophysiological verification) in vitro on K-2 and K-4;
Fig. 3 is a PET image in vivo after the administration of a radioactively labeled K-2 to a Wistar rat;
Fig. 4 is a PET image in vivo after the administration of the radioactively labeled K-2 to a WKY rat;
Fig. 5 is a graph showing the uptake amounts of radioactively labeled K-2 in the brains of the Wistar rat and the WKY rat;
Fig. 6 is a graph showing the result of a forced swimming test in the acute administration of K-2 to the rat;
Fig. 7 is a graph showing the result of the forced swimming test in the acute administration of K-4 to the rat;
Fig. 8 is a graph showing the result of the forced swimming test in the acute administration of K-26 to the rat;
Fig. 9 is a graph showing the result of the forced swimming test in the acute administration of K-30 to the rat;
Fig. 10 is a graph showing the result of the forced swimming test in the acute administration of K-32 to the rat;
Fig. 11 is a graph showing the result of the forced swimming test in the chronic administration of K-2 to the rat;
Fig. 12 is a graph showing the result of the forced swimming test in the chronic administration of K-4 to the rat;
Fig. 13 is a graph showing the result of PET imaging on healthy persons to which [$^{11}$C] K-2 was administered; and
Fig. 14 is PET imaging images of the healthy persons to which [$^{11}$C] K-2 was administered.

PREFERRED MODE FOR CARRYING OUT THE INVENTION

Embodiments of the present invention will be described in detail below.

(1. Definitions)

The term "alkyl" means a monovalent group which is produced when one hydrogen atom of a saturated aliphatic hydrocarbon is lost. An alkyl has, for example, 1 to 15 ($C_1$-$C_{15}$) carbon atoms, and typically has 1 to 10 ($C_1$-$C_{10}$), 1 to 8 ($C_1$-$C_8$), 1 to 6 ($C_1$-$C_6$), 1 to 5 ($C_1$-$C_5$), 1 to 4 ($C_1$-$C_4$), 1 to 3 ($C_1$-$C_3$), 1 to 2 ($C_1$-$C_2$) or 2 to 6 ($C_2$-$C_6$) carbon atoms. An alkyl may be linear or branched. Examples of the alkyl include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, t-butyl, pentyl, isopentyl, neopentyl, hexyl and the like. An alkyl may be further substituted with an appropriate substituent.

The term "alkenyl" means an unsaturated aliphatic hydrocarbon group having at least one double bond. An alkenyl has, for example, 2 to 15 ($C_2$-$C_{15}$) carbon atoms, and typically has 2 to 10 ($C_2$-$C_{10}$), 2 to 8 ($C_2$-$C_8$), 2 to 6 ($C_2$-$C_6$), 2 to 5 ($C_2$-$C_5$), 2 to 4 ($C_2$-$C_4$), 2 to 3 ($C_2$-$C_3$), 3 to 6 ($C_3$-$C_6$), 3 to 8 ($C_3$-$C_8$), 4 to 6 ($C_4$-$C_6$), 4 to 7 ($C_4$-$C_7$) or 4 to 8 ($C_4$-$C_8$) carbon atoms. An alkenyl may be linear or branched. Examples of the alkenyl include, but are not limited to, specifically, vinyl ($-CH=CH_2$), allyl ($-CH_2CH=CH_2$), $-CH=CH(CH_3)$, $-CH=C(CH_3)_2$,$-C(CH_3)=CH_2$, $-C(CH_3)=CH(CH_3)$, $-C(CH_2CH_3)=CH_2$, 1,3-butadienyl ($-CH=CH-CH=CH_2$) , hepta-1,6-diene-4-yl ($-CH_2-(CH_2CH=CH_2)_2$) and the like. An alkenyl may be further substituted with an appropriate substituent.

The term "alkynyl" means an unsaturated aliphatic hydrocarbon group having at least one triple bond. An alkynyl has, for example, 2 to 15 ($C_2$-$C_{15}$) carbon atoms, and typically has 2 to 10 ($C_2$-$C_{10}$), 2 to 8 ($C_2$-$C_8$), 2 to 6 ($C_2$-$C_6$), 2 to 5 ($C_2$-$C_5$), 2 to 4 ($C_2$-$C_4$), 2 to 3 ($C_2$-$C_3$), 3 to 6 ($C_3$-$C_6$), 3 to 8 ($C_3$-$C_8$), 4 to 6 ($C_4$-$C_6$), 4 to 7 ($C_4$-$C_7$) or 4 to 8 ($C_4$-$C_8$) carbon atoms. An alkynyl may be linear or branched. Examples of the alkynyl include, but are not limited to, ethynyl ($-C\equiv CH$),$-C\equiv CH(CH_3)$, $-C\equiv C(CH_2CH_3)$, $-CH_2C\equiv CH$, $-CH_2C\equiv C(CH_3)$, $-CH_2C\equiv C(CH_2CH_3)$ and the like. An alkynyl may be further substituted with an appropriate substituent.

The term "halogen" or "halo" means fluoro (-F), chloro (-Cl), bromo (-Br) and iodine (-I).

The term "pharmaceutically acceptable salt" indicates a salt which is not harmful to mammals, in particular, humans. The pharmaceutically acceptable salt can be formed of a nontoxic acid or base which contains an inorganic acid or base or an organic acid or base. Examples of the pharmaceutically acceptable salt include metal salts formed

of aluminum, calcium, lithium, magnesium, potassium, sodium, zinc and the like and organic salts formed of lysine, N,N'-dibenzylethylenediamine, chloroprocaine, choline, diethanolamine, ethylenediamine, meglumine (N-methylglucamine), procaine and the like. The pharmaceutically acceptable salt contains an acid-addition salt and a base-addition salt.

The term "solvate" means a solvent-containing compound which is formed by association of one or a plurality of solvent molecules with the compound of the present invention. Examples of the solvate include a monosolvate, a disolvate, a trisolvate and a tetrasolvate. The solvate contains a hydrate. Examples of the term "agent" include a medicine, a medical food, food and a supplement. Examples of the "food" include a functional food, a health food, a health oriented food and the like.

(2. Compound)

The present invention provides a prophylactic and/or therapeutic agent for diseases associated with AMPA receptors which contains the compound of formula (I) below or a pharmaceutically acceptable salt or solvate thereof.

[Chem. 4]

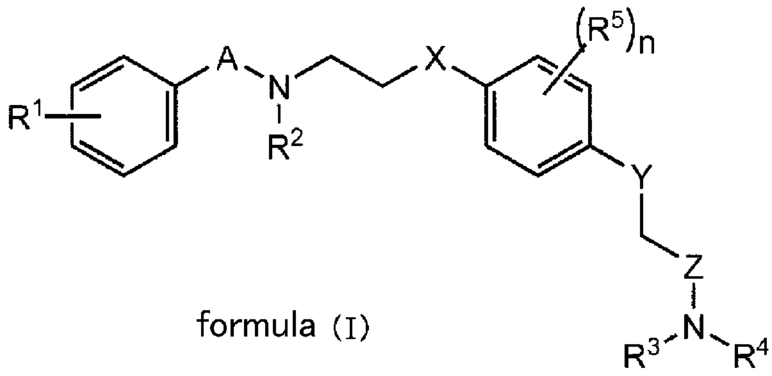

formula (I)

In the formula, each of A and Z independently represents CO, SO or $SO_2$, and in the case of these groups, it is expected that an interaction between the groups and the AMPA receptors is exhibited. Among these, preferably, each of A and Z independently represents CO or $SO_2$, more preferably, A represents $SO_2$ and Z represents CO. Each of X and Y independently represents S or O, preferably, X represents S and Y represents O. Each of $R^1$ to $R^4$ independently represents hydrogen, alkyl, alkenyl, alkynyl or halo. In an embodiment, not all of $R^1$ to $R^4$ are hydrogen, that is, at least one of $R^1$ to $R^4$ represents an element other than hydrogen. Among them, particularly preferably, in terms of therapeutic effects, $R^2$ represents alkyl. In another embodiment, $R^1$ represents alkyl or halo. $R^1$ can be located in any of an ortho-position, a meta-position and a para-position. Preferably, $R^1$ is located in the para-position. In still another embodiment, one of $R^3$ and $R^4$ represents hydrogen and the other represents alkyl. More preferably, each of $R^1$, $R^3$ and $R^4$ independently represents hydrogen, alkyl, alkenyl, alkynyl or halo, and $R^2$ represents alkyl, alkenyl or alkynyl. Each $R^5$ independently represents alkyl, alkenyl, alkynyl or halo. $R^5$ preferably represents halo, particularly preferably fluoro. Further preferably, $R^5$ is located in both ortho-positions with respect to a Y group (that is, both meta-positions with respect to an X group). n represents an integer of 0 to 4. Preferably, n represents 2.

In still another embodiment, as a combination of individual substituents in the compound of formula (I), a combination is preferable in which each of A and Z independently represents CO, SO or $SO_2$, each of X and Y independently represents S or O, each of $R^1$, $R^3$ and $R^4$ independently represents hydrogen, alkyl, alkenyl, alkynyl or halo, $R^2$ represents alkyl, alkenyl or alkynyl, each $R^5$ independently represents alkyl, alkenyl, alkynyl or halo and n represents an integer of 0 to 4.

In still another embodiment, as a combination of individual substituents in the compound of formula (I), a combination is preferable in which A represents $SO_2$, Z represents CO, X represents S, Y represents O, $R^2$ represents alkyl, $R^1$ represents hydrogen, alkyl, or halo, and in a case where $R^1$ represents alkyl or halo, $R^1$ is located in a para-position, one of $R^3$ and $R^4$ represents hydrogen and the other represents alkyl, each $R^5$ independently represents alkyl, alkenyl, alkynyl or halo and n represents an integer of 0 to 4.

In still another embodiment, as a combination of individual substituents in the compound of formula (I), a combination is preferable in which A represents $SO_2$, Z represents CO, X represents S, Y represents O, $R^2$ represents alkyl, $R^1$ represents hydrogen, alkyl, or halo, and in a case where $R^1$ represents alkyl or halo, $R^1$ is located in a para-

position, one of $R^3$ and $R^4$ represents hydrogen and the other represents alkyl, $R^5$ represents halo, in particular, fluoro, $R^5$ is located in both ortho-positions with respect to a Y group (that is, both meta-positions with respect to an X group) and n represents 2.

In still another embodiment, as a combination of individual substituents in the compound of formula (I), a combination is preferable in which A represents $SO_2$, Z represents CO, X represents S, Y represents O, $R^2$ represents alkyl, $R^1$ represents hydrogen, alkyl or halo, and in a case where $R^1$ represents alkyl or halo, $R^1$ is located in a para-position, both of $R^3$ and $R^4$ represent hydrogen, each $R^5$ independently represents alkyl, alkenyl, alkynyl or halo and n represents an integer of 0 to 4.

In still another embodiment, as a combination of individual substituents in the compound of formula (I), a combination is preferable in which A represents $SO_2$, Z represents CO, X represents S, Y represents O, $R^2$ represents alkyl, $R^1$ represents hydrogen, alkyl or halo, and in a case where $R^1$ represents alkyl or halo, $R^1$ is located in a para-position, both of $R^3$ and $R^4$ independently represent alkyl, $R^5$ represents halo, in particular, fluoro, $R^5$ is located in both ortho-positions with respect to a Y group (that is, both meta-positions with respect to an X group) and n represents 2.

In an embodiment, the following may be removed from the compound of formula (I): 4-[2-(4-chlorophenylsulfonylamino) ethylthio]-2,6-difluorophenoxyacetamide, N,N-dimethyl-4-[2-(4-chlorophenylsulfonylamino) ethylthio]-2,6-difluorophenoxyacetamide, 4-[2-(4-chlorophenylsulfonylamino) ethylthio]-2-fluorophenoxyacetamide, N,N-dimethyl-4-[2-(4-chlorophenylsulfonylamino) ethylthio]-2-fluorophenoxyacetamide, 4-[2-(phenylsulfonylamino) ethylthio]-2-fluorophenoxyacetamide and N,N-dimethyl-4-[2- (phenylsulfonylamino) ethylthio]-2-fluorophenoxyacetamide.

Specific examples of the compound of formula (I) include:

[Table 1]

| | Compound Name | Abbreviation | Structural Formula |
|---|---|---|---|
| | {4-[2-(benzenesulfonyl-methyl-amino)-ethylsulfanil]-2,6-difluoro-phenoxy}-acetamide | K-2 | 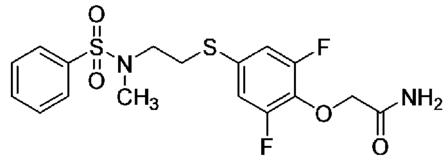 |
| | {4-[2-(benzenesulfonyl-methyl-amino)-ethylsulfanil]-2,6-difluoro-phenoxy}-N,N-dimethyl-acetamide | K-4 | 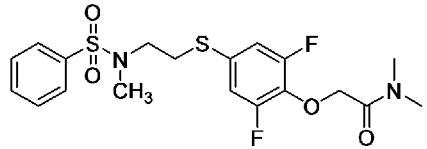 |
| | {4-[2-(benzenesulfonyl-amino)-ethylsulfanil]-2,6-difluoro-phenoxy}-N,N-dimethyl-acetamide | K-5 | 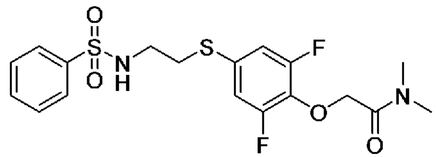 |
| | {4-[2-(benzenesulfonyl-amino)-ethylsulfanil]-2,6-difluoro-phenoxy}-N-methyl-acetamide | M-1 | 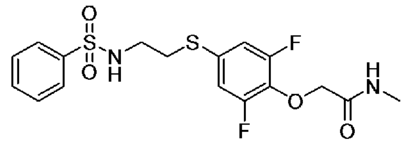 |
| | 2-{2,6-difluoro-4-[2-(4-fluoro-benzenesulfonylamino)-ethylsulfanil]-phenoxy}-acetamide | M-2 | 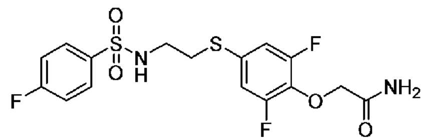 |
| | 2-{2,6-difluoro-4-[2-(4-methyl-benzenesulfonylamino)-ethylsulfanil]-phenoxy}-acetamide | M-3 | 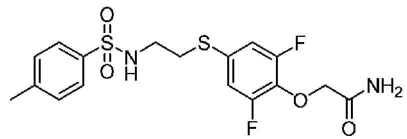 |

(continued)

| | Compound Name | Abbreviation | Structural Formula |
|---|---|---|---|
| | {4-[2-(4-fluoro-benzenesulfonyl-methyl-amino)-ethylsulfanil]-2,6-difluoro-phenoxy}-N,N-dimethyl-acetamide | K-26 | 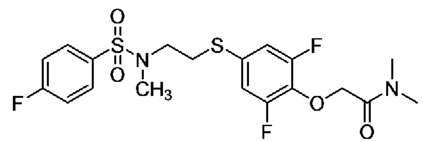 |
| | {4-[2-(benzenesulfonyl-methyl-amino)-ethylsulfanil]-2,6-difluoro-phenoxy}-N,N-diethyl-acetamide | K-30 | 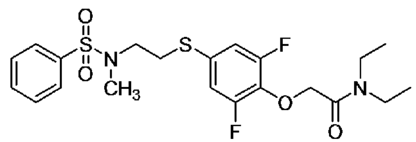 |
| | {4-[2-(benzenesulfonyl-methyl-amino)-ethylsulfanil]-2,6-difluoro-phenoxy}-N-methyl-acetamide | K-32 | 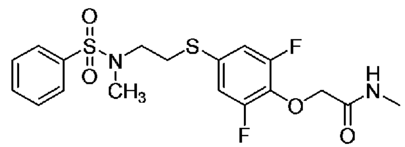 |

Among the typical compounds described above, K-2 and K-4 are preferable, and K-4 is more preferable.

(3. Manufacturing method and intermediate)

(Synthesis example 1)

The compound of formula (I) or the pharmaceutically acceptable salt or solvate thereof in which $R^2$ represents alkyl, alkenyl or alkynyl can be manufactured, for example, by reacting a compound of formula (II) below or a pharmaceutically acceptable salt or solvate thereof (in the formula, A, X, Y, Z, $R^1$, $R^3$, $R^4$, $R^5$ and n are the same as defined in the compound of formula (I)) with $X^1$-$R^2$ (in the formula, $R^2$ represents alkyl, alkenyl or alkynyl and $X^1$ represents halogen):

[Chem. 5]

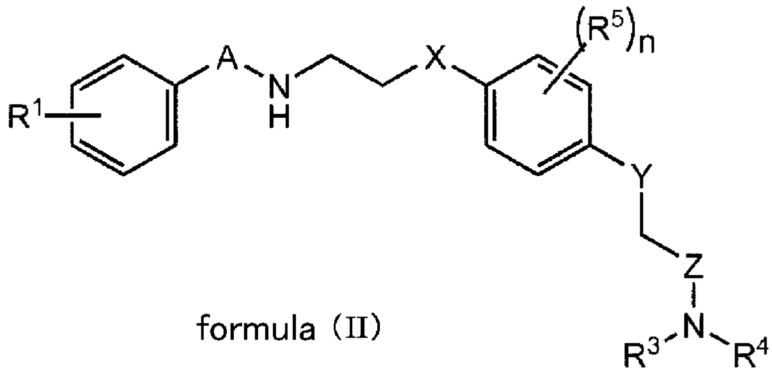

formula (II)

In an embodiment, both $R^3$ and $R^4$ in formula (I) and formula (II) represent hydrogen. In an embodiment, $X^1$ represents I. Specific examples of the compound of formula (II) include 2-[2,6-difluoro-4-({2-[(phenylsulfonyl)amino]ethyl}thio)phenoxy]acetamide (PEPA).

The reaction can be performed in a polar aprotic solvent such as dimethylformamide (DMF), tetrahydrofuran, acetonitrile, acetone or dimethylsulfoxide. The reaction is preferably performed using a base such as NaOH under basic conditions. The reaction temperature is room temperature to reflux temperature, particularly preferably 60 to 100°C and more preferably 80°C. The reaction time is 1 to 10 minutes, and in particular, is 5 minutes.

The present inventors have found that the reaction of the compound of formula (II) with $X^1$-$R^2$ quantitatively occurs in a NH group adjacent to the A group of the compound of formula (II). Hence, even if $R^3$ and $R^4$ represent hydrogen, only the NH group can be converted into an N-$R^2$ group without use of a protecting group. The compound of formula (II) or the pharmaceutically acceptable salt or solvate thereof can be used as an intermediate for manufacturing the compound of formula (I) or the pharmaceutically acceptable salt or solvate thereof in which $R^2$ represents alkyl, alkenyl or alkynyl.

The compound of formula (I) or the pharmaceutically acceptable salt or solvate thereof can be manufactured by a method described in Examples below.

(4. Diseases associated with AMPA receptors)

Since the compound of formula (I) or the pharmaceutically acceptable salt or solvate thereof indicates an AMPA receptor function enhancement action, it is useful as a pharmaceutical based on the action (Jingli Zhang et al., Rev. Neurosci. 2013; 24 (5): 499-505, and Simon E Ward et al., British Journal of Pharmacology (2010), 160, 181-190).

The compound of formula (I) or the pharmaceutically acceptable salt or solvate thereof in the present invention which indicates the AMPA receptor function enhancement action is useful for mammals, in particular, primates such as humans and monkeys as a prophylactic and/or therapeutic agent for diseases such as:

(1) mental diseases such as depression, major depression, bipolar depression, dysthymia, emotional disorder, recurrent depression, postnatal depression, stress disorder, depression symptoms, manic disorder, anxiety, generalized anxiety disorder, anxiety syndrome, panic disorder, phobia, social phobia, social anxiety disorder, obsessive compulsive disorder, post-traumatic stress syndrome, post-traumatic stress disorder, Tourette's syndrome, autism,

fragile X syndrome, Rett syndrome, adjustment disorder, bipolar disorder, neuropathy, schizophrenia, chronic fatigue syndrome, anxiety neurosis, compulsive neurosis, panic disorder, epilepsy, hypersensitivity, attention deficit hyperactivity disorder, psychotic major depression, refractory major depression and treatment resistant depression;
(2) degenerative neurological disorders such as Alzheimer's disease, Alzheimer's-type senile dementia, Parkinson's disease, Huntington's chorea, multiple cerebral infarction dementia, frontotemporal dementia, Parkinson's-type frontotemporal dementia, progressive supranuclear palsy, Pick's syndrome, Niemann-Pick syndrome, corticobasal degeneration, Down syndrome, vascular dementia, Lewy body dementia, amyotrophic lateral sclerosis, motor neurogenic disease, Creutzfeldt-Jakob's disease, cerebral palsy, progressive supranuclear paralysis and multiple sclerosis;
(3) cognitive and memory impairments associated with aging such as age-related memory disorder and senile dementia;
(4) sleep disorders such as intrinsic sleep disorder, extrinsic sleep disorder, circadian rhythm disorder, sleep related disease, sleep disorder associated with internal medicine or psychiatric disorder, stress insomnia, insomnia, insomniac neurosis and sleep apnea syndrome;
(5) respiratory depression caused by anesthetic, traumatic disease or neurodegenerative disease; and
(6) traumatic brain injury, stroke, anorexia, eating disorder, anorexia nervosa, bulimia nervosa, other eating disorders, alcoholism, alcohol abuse, alcohol amnesia, alcoholism, alcohol preference, alcohol withdrawal, alcoholic psychosis, alcohol poisoning, alcoholic jealousy, alcoholic mania, alcohol dependent mental disorder, alcohol psychosis, drug preference, drug phobia, drug mania, drug withdrawal, migraine headache, stress headache, tension headache, diabetic neuropathy, obesity, diabetes, muscle spasms, Meniere's disease, autonomic imbalance, alopecia, glaucoma, deafness, hypertension, heart disease, tachycardia, congestive heart failure, hyperpnea, bronchial asthma, apnea, sudden infant death syndrome, inflammatory disease, allergic disease, impotence, menopause, infertility, cancer, immunodeficiency syndrome due to HIV infection, encephalomyelitis, acromegaly, incontinence, metabolic syndrome, osteoporosis, peptic ulcer, irritable bowel syndrome, inflammatory bowel disease, ulcerative colitis, Crohn's disease, stress gastrointestinal disorder, neurogenic vomiting, peptic ulcer, diarrhea, constipation and postoperative ileus.

Since the compound of formula (I) or the pharmaceutically acceptable salt or solvate thereof in the present invention is excellent in brain delivery, it is particularly useful as a prophylactic and/or therapeutic agent for diseases such as depression and epilepsy.
The compound of formula (I) or the pharmaceutically acceptable salt or solvate thereof in the present invention may be used together with other active ingredients.
Examples of the other active ingredients described above include:
benzodiazepines such as diazepam, tricyclic or tetracyclic antidepressants such as imipramine hydrochloride and desipramine hydrochloride, selective serotonin reuptake inhibitors such as paroxetine hydrochloride, serotonin-noradrenaline reuptake inhibitors such as venlafaxine hydrochloride, noradrenaline reuptake inhibitors such as levoxetine mesylate, 5-$HT_{1A}$ agonists, 5-$HT_3$ antagonists such as siamemazine, histamine $H_1$ antagonist, drugs for treating schizophrenia such as chlorpromazine and clozapine, CRF antagonists, anti-anxiety drugs such as meprobamate, tachykinin antagonists, drugs acting on metabotropic glutamate receptor, CCK antagonist, $\beta$3 adrenergic antagonist, GAT-1 inhibitors, NMDA glycine site agonists, NMDA antagonists, peripheral benzodiazepine receptor agonists, vasopressin antagonists, vasopressin V1b antagonists, vasopressin V1a antagonists, phosphodiesterase inhibitors, opioid antagonists, opioid agonists, nicotinic acid receptor agonists, MAO inhibitors, 5-$HT_{2A}$ antagonists, COMT inhibitors, bipolar disorder treatment drugs such as lithium carbonate, cannabinoid CB1 antagonists, sodium channel inhibitors, anti-ADHD drugs such as methamphetamine hydrochloride, drugs for treating alcoholism, drugs for treating autism, drugs for treating chronic fatigue syndrome, anticonvulsants, drugs for treating insomnia such as triazolam, drugs for treating myasthenia gravis, drugs for treating cerebral infarction, drugs for treating gonorrhea, drug for treating hypersomnia, drugs for treating pain, drugs for treating dysthymia, drugs for treating autonomic dystonia, drugs for treating alcohol-related diseases, drugs for treating irritable bowel syndrome, drugs for treating Alzheimer's disease such as donepezil, drug for treating Parkinson's disease, drugs for treating ALS, drugs for dyslipidemia such as pravastatin sodium, sedatives, apoptosis inhibitors, anti-obesity drugs, anti-diabetic drugs, anti-hypertensive drugs, rheumatic agents, anticancer agents, calcium receptor antagonists, neural differentiation promoters, neural regeneration promoters, non-steroidal antiinflammatory agents, steroids, anti-cytokine drugs, antibody drugs, nucleic acids and nucleic acid derivatives.

(5. Pharmaceutical composition)

The compound of formula (I) or the pharmaceutically acceptable salt or solvate thereof in the present invention can be administered orally or parenterally in order to perform prophylaxis and/or therapy. As an orally administered drug, a solid preparation such as a powder, a granule, a capsule or a tablet or a liquid preparation such as a syrup or an elixir

can be used. As a parenterally administered drug, an injection, preferably an intravenous injection, a rectally administered drug, a skin external preparation or an inhalant can be used. These preparations are manufactured according to a normal method by adding pharmaceutically acceptable manufacturing aids to active ingredients. Furthermore, by a known technology, they can also be formed as sustained preparations.

In order for an orally administered solid preparation to be manufactured, the active ingredient and an excipient such as lactose, starch, crystalline cellulose, calcium lactose, magnesium aluminometasilicate or anhydrous silica are mixed together so as to form a powder or furthermore, as necessary, for example, a binder such as white sugar, hydroxypropyl cellulose or polyvinyl pyrrolidone, a disintegrant such as carboxymethyl cellulose or carboxymethyl cellulose calcium or the like is added and the result is granulated by a wet method or a dry method so as to form a granule. In order for a tablet to be manufactured, the powder or the granule is preferably tableted as it is or by addition of a lubricant such as magnesium stearate or talc. The granule or the tablet can also be coated with an enteric base such as hydroxypropyl methylcellulose phthalate, methacrylic acid or methyl methacrylate copolymer so as to form an enteric preparation or coated with ethyl cellulose, carnauba wax, hardened oil or the like so as to form a sustained preparation. In order for a capsule to be manufactured, the powder or the granule can be charged into a hard capsule or the active ingredient can be dissolved in glycerin, polyethylene glycol, sesame oil, olive oil or the like and then coated with a gelatin film so as to form a soft capsule.

In order for an orally administered liquid preparation to be manufactured, the active ingredient and a sweetener such as white sugar, sorbitol or glycerin may be dissolved in water so as to form an elixir by addition of transparent syrup and furthermore, essential oil, ethanol or the like or so as to form an emulsion or a suspension agent by addition of gum arabic, tragacanth, polysorbate 80, sodium carboxymethylcellulose or the like. As desired, a flavoring agent, a coloring agent, a preservative or the like may be added to the liquid preparations described above.

In order for an injection to be manufactured, the active ingredient may be dissolved in distilled water for injection, as necessary, together with a pH adjuster such as hydrochloric acid, sodium hydroxide, lactose, sodium lactate, sodium monohydrogen phosphate or sodium dihydrogen phosphate and an isotonicity agent such as sodium chloride or glucose and be sterile-filtered so as to be charged into an ampule or may be further subjected to addition of mannitol, dextrin, cyclodextrin, gelatin or the like and be lyophilized under vacuum so as to form a dissolution-type injection. The active ingredient can be emulsified in water by addition of lecithin, polysorbate 80, polyoxyethylene hardened castor oil or the like so as to form an emulsion for injection.

Preferably, in order for a rectally administered drug to be manufactured, the active ingredient and a suppository base such as cocoa butter, tri-, di- and mono-glycerides of a fatty acid or polyethylene glycol are humidified and melted and are poured into a mold so as to be cooled or the active ingredient is dissolved in polyethylene glycol, soybean oil or the like and then coated with a gelatin film.

In order for a skin external preparation to be manufactured, the active ingredient is added to white petrolatum, beeswax, liquid paraffin, polyethylene glycol or the like and is humidified and mixed as necessary so as to form an ointment or is mixed with an adhesive such as rosin or acrylic acid alkyl ester polymer and is thereafter spread over a non-woven fabric such as polyethylene so as to form a tape agent. In order for an inhalant to be manufactured, the active ingredient is dissolved or dispersed in a propellant such as Freon gas and is charged into a pressure-resistant container so as to form an aerosol agent.

The prophylactic and/or therapeutic agent in the present invention configured as described above can be manufactured by a known manufacturing method such as a method described in the general rules for preparation in The Japanese Pharmacopoeia 10th edition or a method obtained by appropriately improvement thereof.

Although the administered amount of prophylactic and/or therapeutic agent in the present invention differs depending on the age, the weight and the condition of a patient, the administered amount is normally about 1 to 1000 mg per day equivalent to the compound of formula (I) or the pharmaceutically acceptable salt or solvate thereof, and is preferably divided into one or several doses so as to be administered.

## EXAMPLES

Examples will be described below. The examples discussed below are described in order to deeply understand the scope of the claims of the present invention, and are not intended to limit the scope of the claims of the present invention.

(Manufacturing example 1)

(Synthesis of K-1, K-2, K-4 and K-5)

2-[2,6-difluoro-4-({2-[(phenylsulfonyl)amino]ethyl}thio)phenoxy]acetamide (K-1, PEPA) and {4-[2-(benzenesulfonyl-methyl-amino)-ethylsulfanil]-2,6-difluoro-phenoxy}-acetamide (K-2) were synthesized according to the following

scheme. The $^1$H NMR spectrum of each compound was recorded with Bruker Avance III 400 MHz or Varian Mercury plus-300 MHz by using TMS as an internal reference.

[Chem. 6]

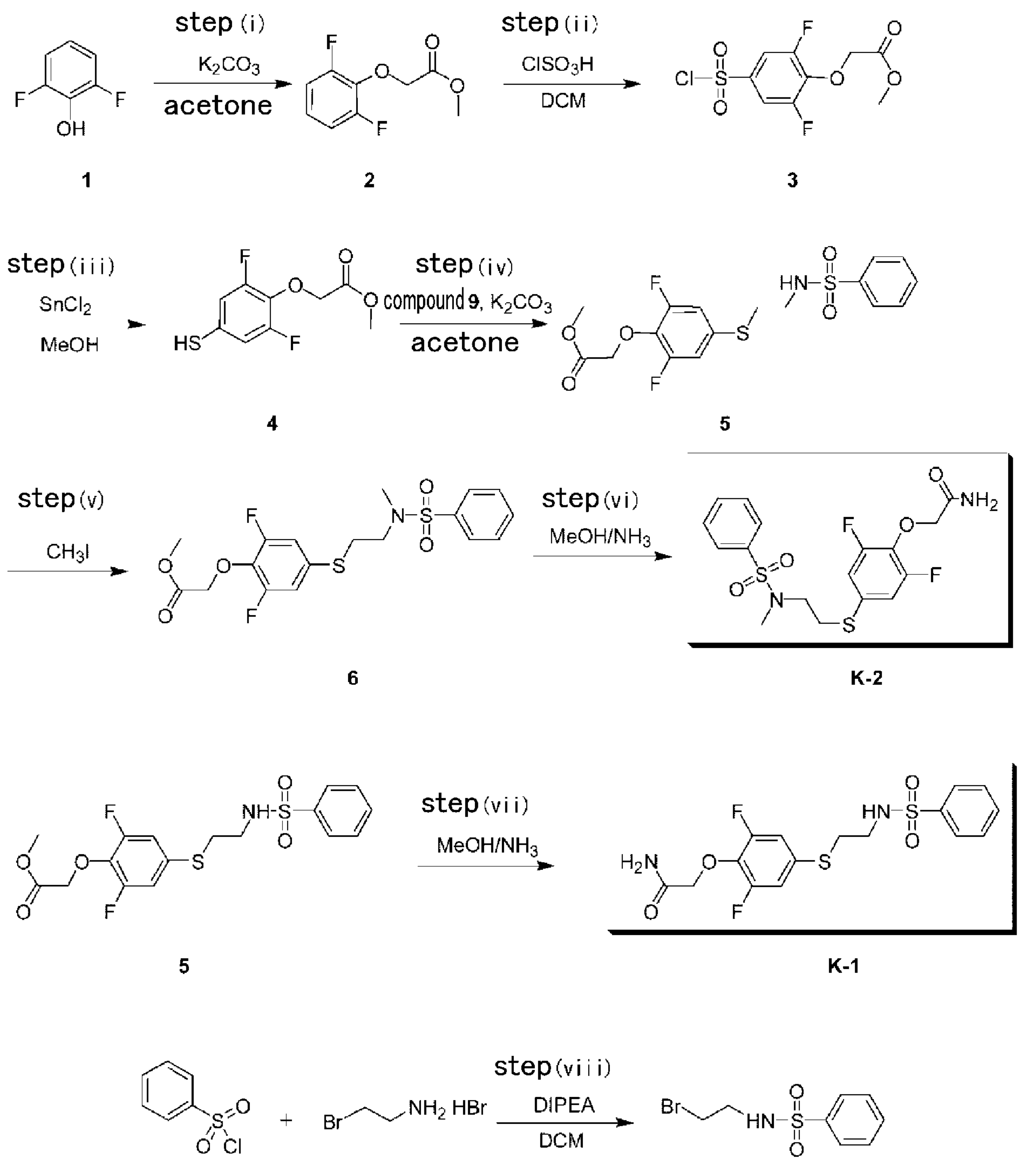


1 2 3 4 5 6 K-2 5 K-1 7 8 9

Step (i): Synthesis of (2,6-difluoro-phenoxy)-acetic acid methyl ester (2)

[Chem. 7]

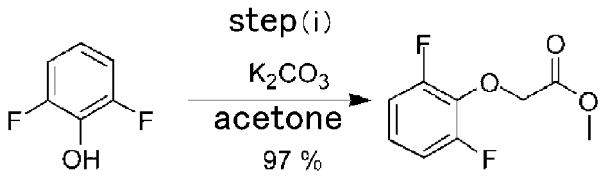


1 2

To an acetone solution (75 mL) of 2,6-difluoro-phenol (1) (5.00 g, 38.5 mmol), $K_2CO_3$ (8.40 g, 60.7 mmol) was added, and after 10 minutes, methyl bromoacetate (5.80 g, 38.5 mmol) was added to the reaction solution. The reaction solution was stirred at room temperature overnight. After the completion of the reaction, the reaction mixture solution was poured to a mixture solution of concentrated hydrochloric acid (20 mL) and ice water (200 ml), the resultant product was extracted with EtOAc (100 mL × 3), an organic layer was washed with water (50 mL × 3) and brine (100 mL × 2), was dried with $Na_2SO_4$ and was filtered. Thereafter, the resultant product was concentrated under vacuum, and thus a compound (2) was obtained as a yellow oil (7.50 g, 97%).
$^1H$ NMR (300 MHz, $CDCl_3$) : δ 3.78 (s, 3H), 4.74 (s, 2H), 6.86-6.99 (m, 3H).

Step (ii): Synthesis of (4-chlorosulfonyl-2,6-difluoro-phenoxy)-acetic acid methyl ester (3)

[Chem. 8]

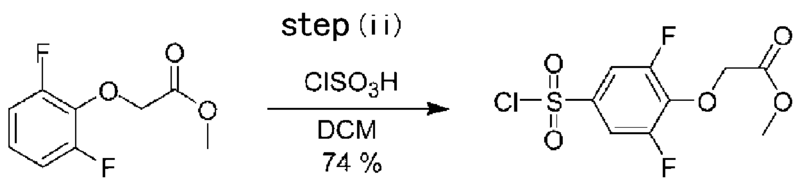


2 3

To a DCM solution of (2,6-difluoro-phenoxy)-acetic acid methyl ester (2) (5.00 g, 24.7 mmol), chlorosulfonic acid (17.2 g, 24.7 mmol) was added dropwise in an ice bath, and the reaction solution was heated to 45°C and was stirred for 1.5 hours. After the completion of the reaction, the reaction mixture solution was quenched with 50 mL of ice water, an organic layer was separated and washed with water (300 mL × 3). The resultant product was dried with $Na_2SO_4$ and was filtered and was then concentrated under vacuum, and thus a compound (3) was obtained as a yellow oil (5.50 g, 74%).
$^1H$ NMR (300 MHz, $CDCl_3$) :δ 3.81 (s, 3H), 4.96 (s, 2H), 7.61 (s, 1H), 7.64 (s, 1H).

Step (iii): Synthesis of (2,6-difluoro-4-mercapto-phenoxy)-acetic acid methyl ester (4)

[Chem. 9]

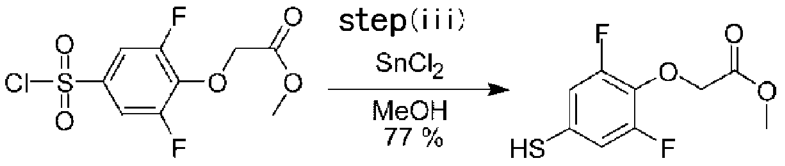


3 4

To a mixture solution of (4-chlorosulfonyl-2,6-difluoro-phenoxy)-acetic acid methyl ester (3) (5.50 g, 18.3 mmol), $SnCl_2$ (14.5 g, 64.2 mmol) and methanol (50 mL), concentrated hydrochloric acid (25 mL) was added dropwise. The reaction mixture solution was heated to reflux temperature and was stirred for 2 hours. After cooling, the reaction mixture solution was poured to ice water (100 mL) and the resultant product was extracted with DCM (100 mL × 3). An organic layer was washed with water (100 mL × 3) and brine (100 mL × 2), was dried with $Na_2SO_4$, was filtered and was then concentrated under vacuum, and thus a compound (4) was obtained as a yellow oil (3.30 g, 77%).
$^1$H NMR (300 MHz, $CDCl_3$) : δ 3.52 (s, 1H), 3.77 (s, 3H), 4.71 (s, 2H), 6.83 (s, 1H), 6.86 (s, 1H).

Step (iv): Synthesis of [4-(2-benzenesulfonylamino-ethylsulfanil)-2,6-difluoro-phenoxy]-acetic acid methyl ester (5)

[Chem. 10]

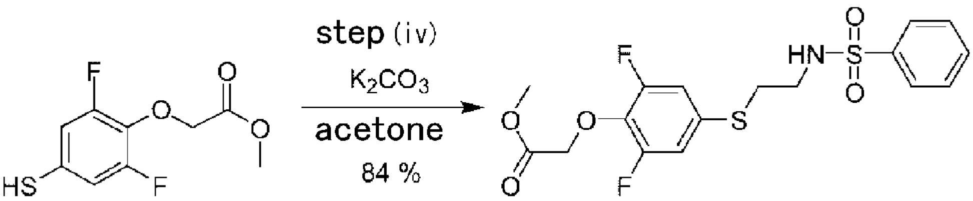


4 5

A mixture solution of (2,6-difluoro-4-mercapto-phenoxy)-acetic acid methyl ester (4) (1.10 g, 4.7 mmol), potassium carbonate (778 mg, 5.6 mmol) and acetone (15 mL) was stirred under $N_2$ at room temperature for 20 minutes. To the reaction solution, N-(2-bromo-ethyl)-benzenesulfonamide (9) (1.30 g, 4.90 mmol) was added, and the reaction solution was stirred at room temperature overnight. After the completion of the reaction, the reaction solution was poured to 30 mL of 2N HCl and the resultant product was extracted with EtOAc (50 mL × 3). An organic layer was washed with water (50 mL × 3) and brine (100 mL × 2), was dried with $Na_2SO_4$, was filtered and was then concentrated under vacuum, and thus a residue was obtained. The residue was refined by silica gel column chromatography (PE/EA = 10/1 to 3/1, v/v), and thus a compound (5) was obtained as a yellow oil (1.60 g, 84%).
$^1$HNMR (300 MHz, $CDCl_3$) : δ 2.95 (t, J = 6.6 Hz, 2H), 3.12 (q, J = 6.3 Hz, 2H), 3.78 (s, 3H), 4.72 (s, 2H), 5.20 (t, J = 6.0 Hz, 1H), 6.76-6.83 (m, 2H), 7.47-7.60 (m, 3H), 7.82-7.84 (m, 2H).

Step (v): Synthesis of {4-[2-(benzenesulfonyl-methyl-amino)-ethylsulfanil]-2,6-difluoro-phenoxy}-acetic acid methyl ester (6)

[Chem. 11]

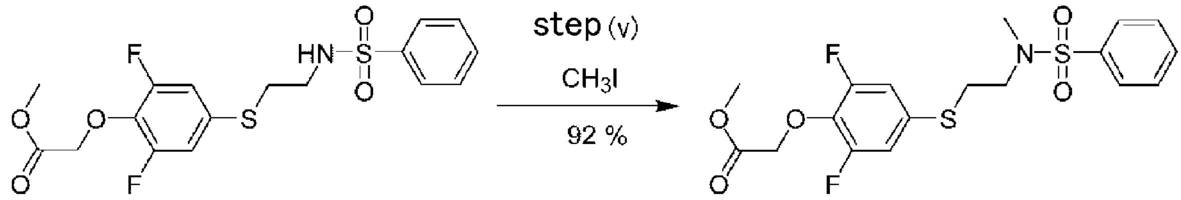


5

6

To 10 mL of a DMF mixture solution of [4-(2-benzenesulfonylamino-ethylsulfanil)-2,6-difluoro-phenoxy]-acetic acid methyl ester (5) (300 mg, 0.72 mmol) and $K_2CO_3$ (397 mg, 2.88 mmol), MeI (255 mg, 1.80 mmol) was added at 0°C. Thereafter, the reaction solution was stirred at room temperature for 1 hour. After the completion of the reaction, the reaction solution was diluted with 20 ml of water, and the resultant product was extracted with EtOAc (30 mL × 3). An organic layer was washed with water (30 mL × 3) and brine (20 mL × 2), was dried with $Na_2SO_4$, was filtered and was then concentrated under vacuum, and thus a compound (6) was obtained as a yellow oil (285 mg, 92%).
$^1$HNMR (300 MHz, $CDCl_3$) : δ 2.81 (s, 3H), 3.04-3.09 (m, 2H), 3.19-3.24 (m, 2H), 3.79 (s, 3H), 4.74 (s, 2H), 6.90-6.94 (m, 2H), 7.50-7.60 (m, 3H), 7.74-7.77 (m, 2H).

Step (vi): Synthesis of {4-[2-(benzenesulfonyl-methyl-amino)-ethylsulfanil]-2,6-difluoro-phenoxy}-acetamide (K-2)

[Chem. 12]

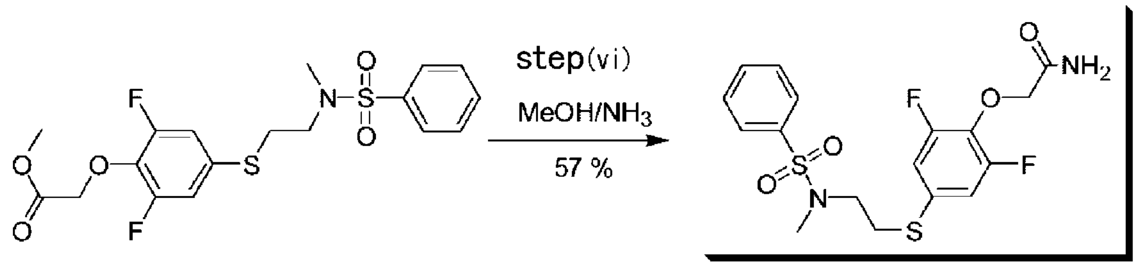


6

K-2

A mixture solution of {4-[2-(benzenesulfonyl-methyl-amino)-ethylsulfanil]-2,6-difluoro-phenoxy}-acetic acid methyl ester (6) (40.0 mg, 0.09 mmol) and 13 mL of 4N MeOH/$NH_3$ was stirred at room temperature for 18 hours. After the completion of the reaction, the reaction mixture solution was concentrated under vacuum, and thus a residue was obtained. The residue was refined by preparative HPLC, and thus a compound (K-2) was obtained as a white solid (22.0 mg, 57%). $^1$HNMR (300 MHz, CDCl3): δ 2.82 (s, 3H), 3.08-3.13 (m, 2H), 3.20-3.26 (m, 2H), 4.58 (s, 2H), 6.93-6.99 (m, 2H), 7.50-7.63 (m, 3H), 7.75-7.78 (m, 2H).

Synthesis of {4-[2-(benzenesulfonyl-methyl-amino)-ethylsulfanil]-2,6-difluoro-phenoxy}-N,N-dimethyl-acetamide (K-4)

[Chem. 13]

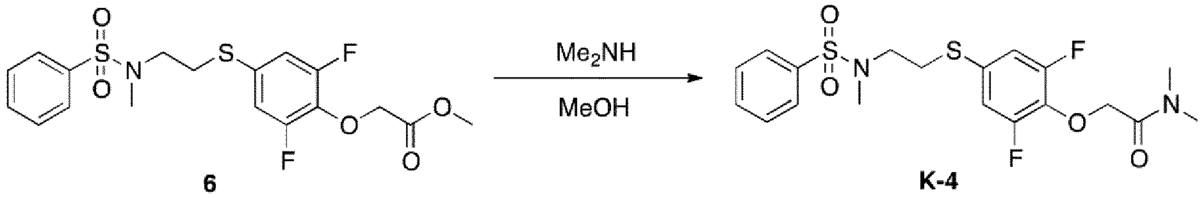


6 K-4

A mixture solution of {4-[2-(benzenesulfonyl-methyl-amino)-ethylsulfanil]-2,6-difluoro-phenoxy}-acetic acid methyl ester (6) (9 g, 27.7 mmol) and 240 mL of 5N dimethylamine/methanol was stirred at room temperature for 2 hours. After the completion of the reaction, the mixture solution was concentrated under reduced pressure and was refined by silica gel chromatography, and thus a compound (K-4) was obtained as a colorless oily substance (12 g, 97%). $^1$HNMR ($CDCl_3$, 400 MHz): δ 2.82 (s, 3H), 2.98 (s, 3H), 3.05-3.09 (m, 5H), 3.19-3.22 (m, 2H), 4.80 (s, 2H), 6.89-6.92 (m, 2H), 7.53-7.55 (m, 2H), 7.58-7.60 (m, 1H), 7.75-7.77 (m, 2H). LCMS [mobile phase: analysis for 6.5 minutes with 55% water (0.05% formic acid) and 45% acetonitrile (0.05% formic acid)] purity > 95%, holding time = 3.445 minutes; MS Calcd.: 444.5; MS Found: 445.0 [M+1]$^+$.

Step (vii): Synthesis of 2-[2,6-difluoro-4-({2-[(phenylsulfonyl)amino]ethyl}thio)phenoxy]acetamide (K-1)

[Chem. 14]

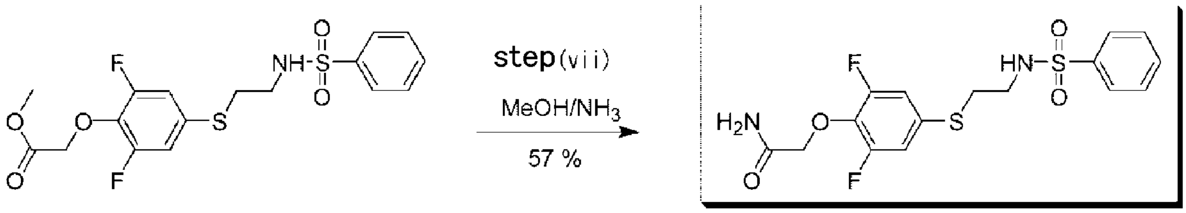


5 K-1

A mixture solution of [4-(2-benzenesulfonylamino-ethylsulfanil)-2,6-difluoro-phenoxy]-acetic acid methyl ester (5) (200 mg, 0.48 mmol) and 10 mL of 4N MeOH/$NH_3$ was stirred at room temperature for 18 hours. After the completion of the reaction, the reaction mixture solution was concentrated under vacuum, and thus a residue was obtained. The residue was refined by preparative HPLC, and thus a compound K-1 was obtained as a white solid (110 mg, 57%). $^1$HNMR (300 MHz, $CDCl_3$ + $D_2O$) : δ 2.97-3.02 (m, 2H), 3.11-3.16 (m, 2H), 4.56 (s, 2H), 6.82-6.90 (m, 2H), 7.48-7.61 (m, 3H), 7.82-7.87 (m, 2H).

Synthesis of {4-[2-(benzenesulfonyl-amino)-ethylsulfanil]-2,6-difluoro-phenoxy}-N,N-dimethyl-acetamide (K-5)

[Chem. 15]

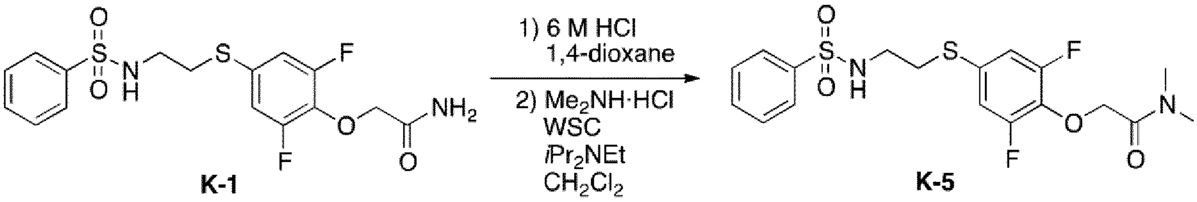


K-1 K-5

K-1 (159.4 mg, 0.396 mmol) was dissolved in 1,4-dioxane (3 mL), was subjected to addition of 6 M hydrochloric acid (1 mL) and was heated at 80°C for 2 hours. The reaction was stopped by addition of water, the resultant product was extracted with ethyl acetate, then a saturated sodium bicarbonate aqueous solution was added to the ethyl acetate solution, the product was extracted in a water layer, hydrochloric acid was added again so as to make the product acidic, then the product was extracted again with ethyl acetate and was washed with saturated saline and thereafter water was removed by addition of anhydrous sodium sulfate. The solution was evaporated under reduced pressure, a residue obtained was dissolved in a dichloromethane solution, dimethylamine · hydrochloride (94.5 mg), WSC · HCl (150.8 mg) and diisopropylethylamine (265 μL) were added to this solution and the solution was stirred at room temperature for 2 hours. The reaction was stopped by addition of hydrochloric acid thereto, and the resultant product was extracted with ethyl acetate. This was washed with a saturated sodium bicarbonate aqueous solution and saturated saline, and the solvent was evaporated under reduced pressure. A residue was refined by silica gel chromatography, and thus target K-5 was obtained as a white solid (148.1 mg, 84%).

Step (viii): Synthesis of N-(2-bromo-ethyl)-benzenesulfonamide (9)

[Chem. 16]

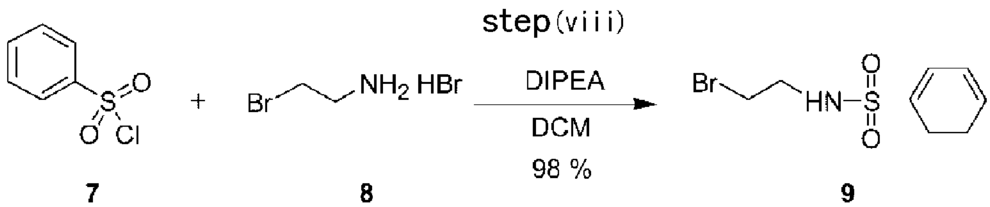


7 8 9

To a DCM (30 mL) solution of benzenesulfonyl chloride (7) (3.00 g, 17.0 mmol) and 2-bromoethylamine hydrobromide (8) (3.80 g, 18.7 mmol), DIPEA (4.80 g, 37.4 mmol) was added in an ice bath. Thereafter, the reaction solution was stirred at the same temperature for 1.5 hours. After the completion of the reaction, the reaction solution was diluted with 20 mL of water, and the resultant product was extracted with EtOAc (30 mL × 3). An organic layer was washed with water (30 mL × 3) and brine (20 mL × 2), was dried with $Na_2SO_4$, was filtered and was then concentrated under vacuum, and thus a compound (9) was obtained as a white solid (4.40 g, 98%).
$^1$HNMR (300 MHz, $CDCl_3$) : δ 3.36-3.39 (m, 4H), 5.09 (s, 1H), 7.50-7.63 (s, 3H), 7.87-7.89 (s, 2H).

(Manufacturing example 2)

(Synthesis of M-1, M-2 and M-3)

According to the following scheme, 2-[4-(2-benzenesulfonylamino-ethylsulfanil)-2,6-difluoro-phenoxy]-N-methyl-acetamide (M-1), 2-{2,6-difluoro-4-[2-(4-fluoro-benzenesulfonylamino)-ethylsulfanil]-phenoxy}-acetamide (M-2) and 2-{2,6-difluoro-4-[2-(4-methyl-benzenesulfonylamino)-ethylsulfanil]-phenoxy}-acetamide (M-3) were synthesized. The $^1$H NMR spectrum of each compound was recorded with Varian Mercury plus-400 MHz by using TMS as an internal reference. The following was used as LCMS: Agilent 1200A, column: C18; column size: 4.6 * 50 minutes; mobile phase: B (ACN), A (water of 0.05% $NH_3$); gradient (B%): as described in Example.

[Chem. 17]

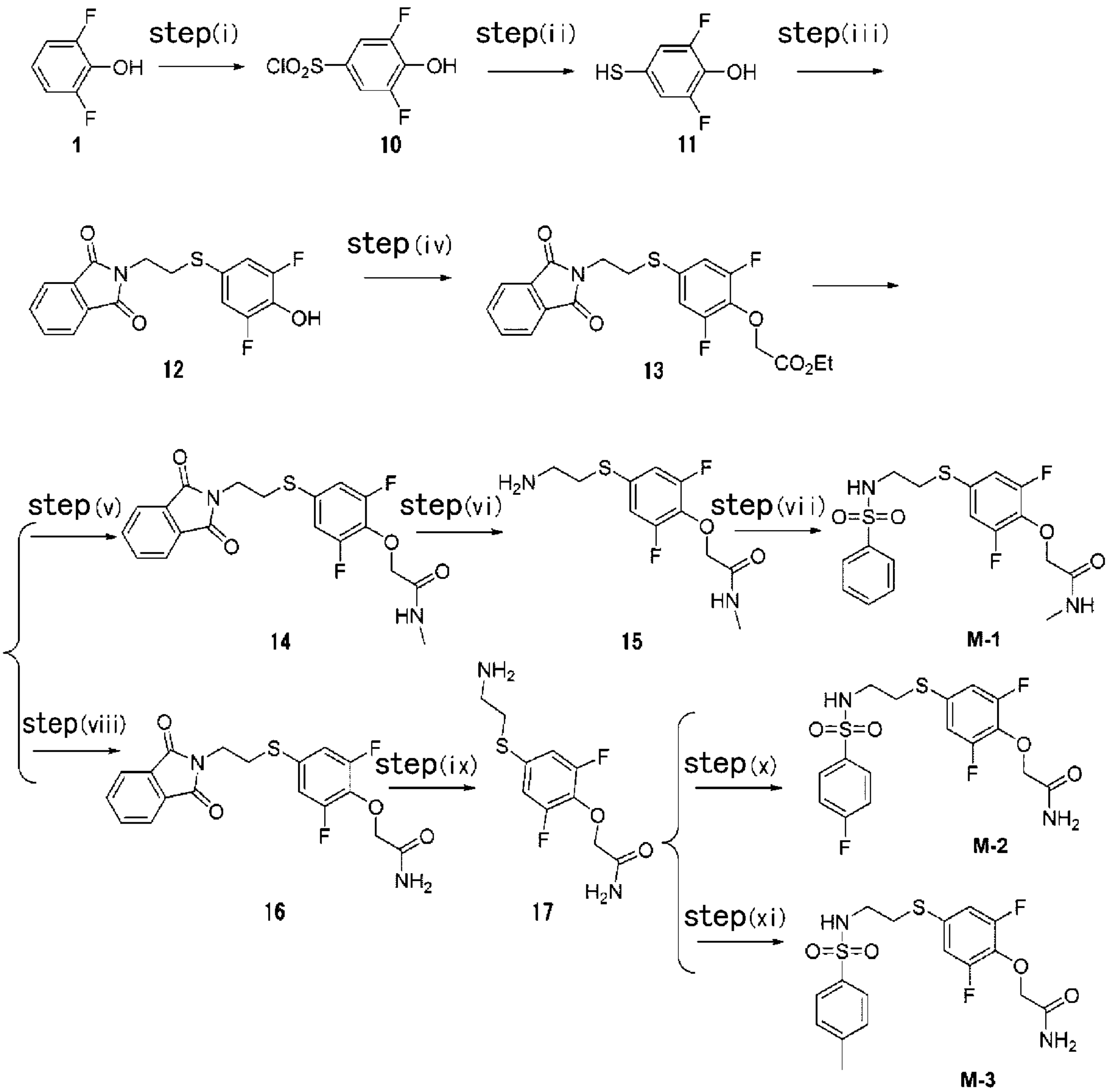


1 10 11 12 13 14 15 M-1 16 17 M-2 M-3

Step (i): Synthesis of 3,5-difluoro-4-hydroxy-benzenesulfonyl chloride (10)

[Chem. 18]

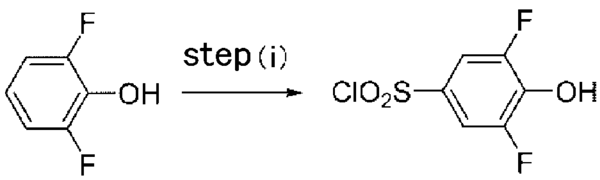


1 10

To a DCM (50 mL) solution of the compound (1) (5.0 g), chlorosulfonic acid (15 mL) was added dropwise. The reaction mixture solution was stirred at 25°C for 1 hour. The TLC (petroleum ether/EtOAc: 20/1) indicated the completion of the reaction. Thereafter, the solution was poured to crushed ice. An organic layer was separated and filtered through Celite. The filtrate was dried and evaporated under vacuum, and thus a compound (10) was obtained as a yellow oil: 5

g (57%).
$^1$H-NMR (400 MHz, $CDCl_3$) : δ 6.30 (s, 1H), 7.66-7.68 (m, 2H) .

Step (ii): Synthesis of 2,6-difluoro-4-mercapto-phenol (11)

[Chem. 19]

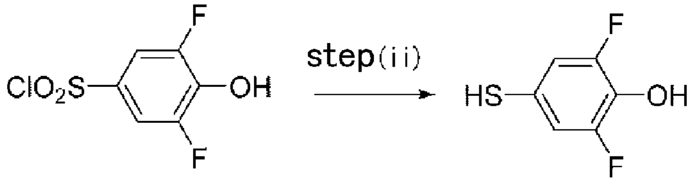


10 11

To a DCM (3 mL) solution of triphenyl phosphine (3.4 g, 13.1 mmol) and DMF (0.1 mL), a DCM (4 mL) solution of the compound (10) (1.0 g, 4.3 mmol) was added dropwise at 0°C under nitrogen. The reaction mixture solution was stirred at 25°C for 2 hours. Thereafter, 1N HCl was added to the mixture solution so as to adjust the pH to 3, and the resultant product was extracted with EA. An organic layer was dried with sodium sulfate to remove the solvent, and thus a crude compound (11) was obtained as a yellow oil.

Step (iii): Synthesis of 2-[2-(3,5-difluoro-4-hydroxy-phenylsulfanyl)-ethyl]-isoindole-1,3-dione (12)

[Chem. 20]

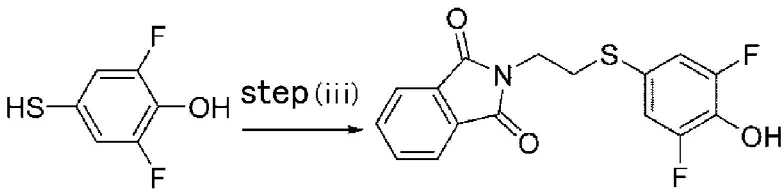


11 12

To a DMF (100 mL) solution of the crude compound (11) (14 g, 86 mmol), 2-(2-bromo-ethyl)-isoindole-1,3-dione (13.2 g, 51.8 mmol) and $K_2CO_3$ (23.8 g, 172.4 mmol) were added. The mixture solution was stirred at 25°C overnight. Thereafter, 1N HCl was added to the mixture solution to adjust the pH to 3, and the resultant product was extracted with EA. An organic layer was dried with sodium sulfate to remove the solvent, and thus a compound (12) was obtained as a yellow solid (8 g, 27%). $^1$H-NMR (400 MHz, DMSO-d6): δ 3.20-3.23 (t, 2H), 3.75-3.79 (t, 2H), 7.08-7.10 (d, 2H), 7.84 (s, 4H).

Step (iv): Synthesis of {4-[2-(1,3-dioxo-1,3-dihydro-isoindole-2-yl)-ethylsulfanil]-2,6-difluoro-phenoxy}-ethyl acetic acid ester (13)

[Chem. 21]

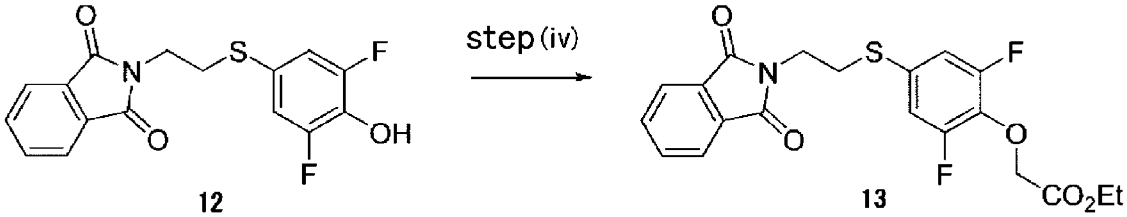


12 13

To a solution obtained by dissolving the compound (12) (5.0 g, 15 mmol) in DMF (30 mL), 3-bromo-propionic acid ethyl ester (2.5 g, 15 mmol) and $K_2CO_3$ (3.0 g, 22.5 mmol) were added. The mixture solution was stirred at 25°C overnight. Thereafter, the mixture solution was subjected to extraction using EA. An organic layer was dried with sodium sulfate to remove the solvent, and thus a compound (13) was obtained as a white solid (6 g, 97%).

$^1$H-NMR (400 MHz, $CDCl_3$) : $\delta$ 1.21-1.24 (t, 3H), 3.11-3.14 (t, 2H), 3.84-3.88 (t, 2H), 4.18-4.20 (d, 2H), 4.61 (s, 2H), 6.91-6.94 (d, 2H), 7.66-7.68 (m, 2H), 7.77-7.79 (m, 2H).

Step (v): Synthesis of 2-{4-[2-(1,3-dioxo-1,3-dihydro-isoindole-2-yl)-ethylsulfanil]-2,6-difluoro-phenoxy}-N-methyl-acetamide (14)

[Chem. 22]

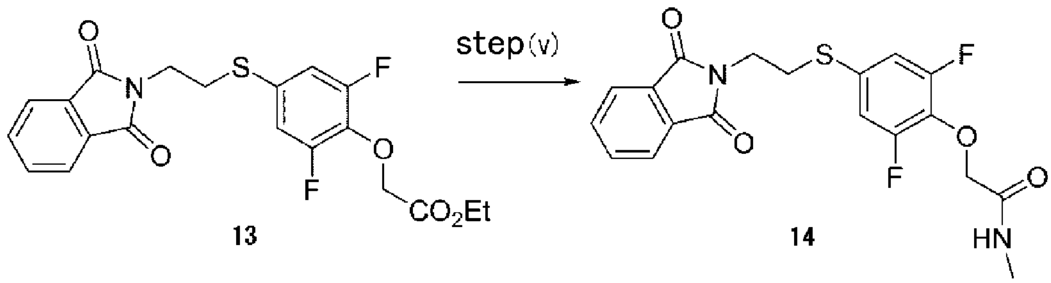


13 14

A methylamine alcohol solution (10 mL) of the compound (13) (0.5 g, 1.2 mmol) was stirred at 100°C for 30 minutes. Thereafter, the mixture solution was concentrated, and thus a crude compound (14) was obtained as a yellow oil (1 g).

Step (vi): Synthesis of 2-[4-(2-amino-ethylsulfanil)-2,6-difluoro-phenoxy]-N-methyl-acetamide (15)

[Chem. 23]

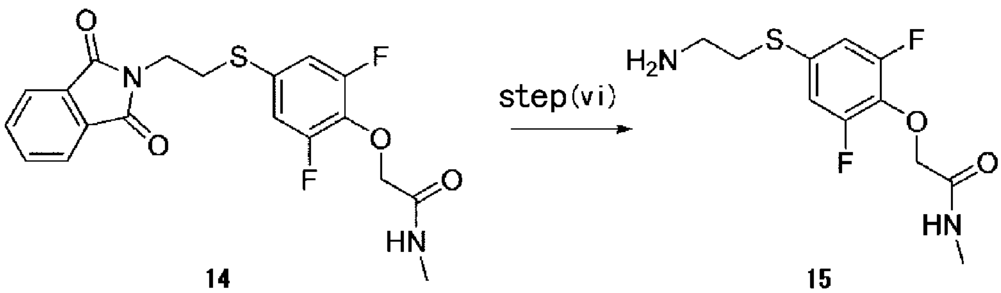


14 15

Hydrazine hydrate (0.25 g, 5 mmol) was added to an EtOH (10 mL) solution of the crude compound (14) (1 g, 2.5 mmol) at 90°C. The solution was heated to 90°C, was stirred for 30 minutes and was then cooled to room temperature. The resultant product was filtered and washed with EtOH. An organic layer was dried with sodium sulfate and was concentrated, and thus a crude compound (15) was obtained as a yellow oil (0.5 g).

Step (vii): Synthesis of 2-[4-(2-benzenesulfonylamino-ethylsulfanil)-2,6-difluoro-phenoxy]-N-methyl-acetamide (M-1)

[Chem. 24]

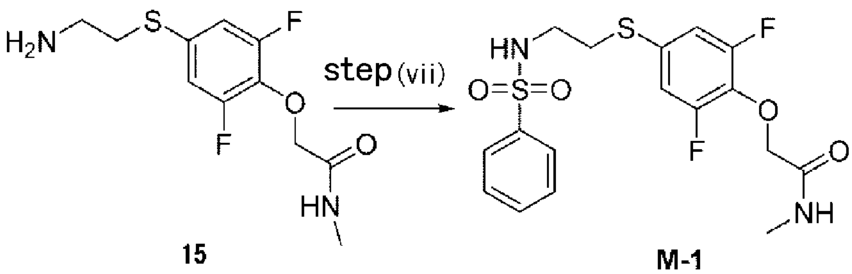


15 M-1

Benzenesulfonyl chloride (0.4 g, 2.2 mmol) and triethylamine (0.2 g, 2.2 mmol) were added to a DCM (10 mL) solution of the crude compound (15) (0.5 g, 1.8 mmol). Thereafter, the mixture solution was stirred at 25°C for 1 hour and was extracted with EA. An organic layer was dried with sodium sulfate and was concentrated. The residue was refined by flash chromatography, and thus a compound (M-1) was obtained as a white solid (20 mg).

$^1$H-NMR (400 MHz, DMSO-d6): δ 2.65-2.66 (d, 3H), 2.91-2.94 (t, 2H), 2.01-3.04 (t, 2H), 4.50 (s, 2H), 7.10-7.12 (d, 2H), 7.57-7.65 (m, 3H), 7.76-7.78 (d, 2H), 7.92-7.95 (t, 1H), 8.05 (s, 1H). MS: m/z 417 $(M+1)^+$

LCMS [mobile phase: 5% water (0.1% $NH_4OH$) and 95% $CH_3CN$ from 90% water (0.1% $NH_4OH$) and 10% $CH_3CN$, 6.0 minutes, finally 0.5 minutes under these conditions] purity 97.4%, Rt = 3.341 minutes; MS Calcd.: 416; MS Found: 417 ($[M+1]^+$).

Step (viii): Synthesis of 2-{4-[2-(1,3-dioxo-1,3-dihydro-isoindole-2-yl)-ethylsulfanil]-2,6-difluoro-phenoxy}-acetamide (16)

[Chem. 25]

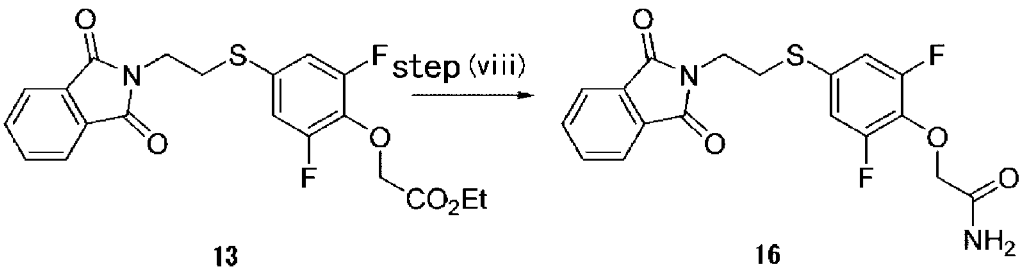


13 16

An $NH_3$/EtOH (100 mL) solution of the compound (13) (5.0 g, 11.8 mmol) was stirred at 25°C for 2 hours. Thereafter, the solution was concentrated, and thus a crude compound (16) was obtained as a yellow oil (6.0 g).

Step (ix): Synthesis of 2-[4-(2-amino-ethylsulfanil)-2,6-difluoro-phenoxy]-acetamide (17)

[Chem. 26]

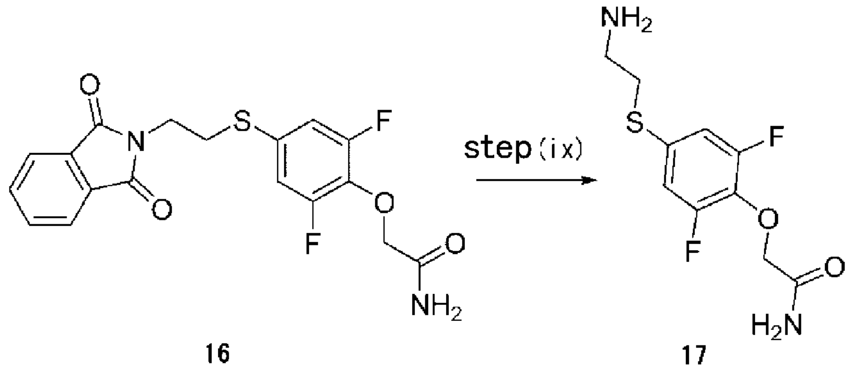


16 17

Hydrazine hydrate (1.5 g, 30 mmol) was added to an EtOH (50 mL) solution of the crude compound (16) (6.0 g, 15.3 mmol) at 90°C. The solution was heated to 90°C, was stirred for 30 minutes and was then cooled to room temperature. The resultant product was filtered and washed with EtOH. An organic layer was dried with sodium sulfate and was concentrated, and thus a crude compound (17) was obtained as a yellow oil (4.0 g).

Step (x): Synthesis of 2-{2,6-difluoro-4-[2-(4-fluoro-benzenesulfonylamino)-ethylsulfanil]-phenoxy}-acetamide (M-2)

[Chem. 27]

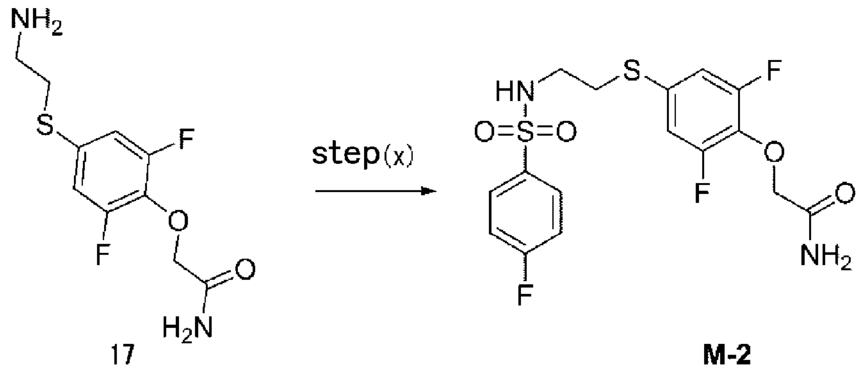


17 **M-2**

4-fluoro-benzenesulfonyl chloride (0.4 g, 2.3 mmol) and triethylamine (0.2 g, 2.2 mmol) were added to a DMF (10 mL) solution of the crude compound 17 (0.5 g, 1.9 mmol). Thereafter, the mixture solution was stirred at 25°C for 1 hour, and the resultant product was extracted with EA. An organic layer was dried with sodium sulfate and was concentrated. The residue was refined by flash chromatography, and thus a compound (M-2) was obtained as a white solid (20 mg) .

$^1$H-NMR (400 MHz, DMSO-d6): $\delta$ 2.92-2.95 (t, 2H), 3.01-3.04 (t, 2H), 4.45 (s, 2H), 7.09-7.11 (d, 2H), 7.40-7.44 (m, 3H), 7.47 (s, 1H), 7.81-7.85 (m, 2H), 7.95-7.98 (t, 1H). MS: m/z 421 $(M+1)^+$

LCMS [mobile phase: 5% water (0.1% $NH_4OH$) and 95% $CH_3CN$ from 90% water (0.1% $NH_4OH$) and 10% $CH_3CN$, 6 minutes, finally 0.5 minutes under these conditions] purity 95.1%, Rt = 3.284 minutes; MS Calcd.: 420; MS Found: 421 ($[M+1]^+$).

Step (xi): Synthesis of 2-{2,6-difluoro-4-[2-(4-methyl-benzenesulfonylamino)-ethylsulfanil]-phenoxy}-acetamide (M-3)

[Chem. 28]

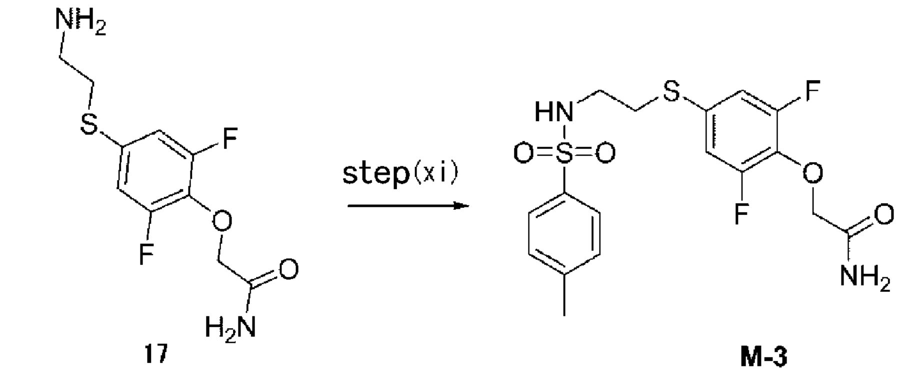


17

M-3

4-methyl-benzenesulfonyl chloride (0.5 g, 2.3 mmol) and triethylamine (0.2 g, 2.2 mmol) were added to a DMF (10 mL) solution of the crude compound (17) (0.5 g, 1.9 mmol). Thereafter, the mixture solution was stirred at 25°C for 1 hour, and the resultant product was extracted with EA. An organic layer was dried with sodium sulfate and was concentrated. The residue was refined by flash chromatography, and thus a compound (M-3) was obtained as a white solid (20 mg) .

$^1$H-NMR (400 MHz, DMSO-d6): δ 2.38 (s, 3H), 2.88-2.91 (t, 2H), 2.99-3.02 (t, 2H), 4.49 (s, 2H), 7.08-7.10 (d, 2H), 7.37-7.48 (m, 4H), 7.64-7.66 (d, 2H), 7.81-7.84 (t, 1H). MS: m/z 417 $(M+1)^+$

LCMS [mobile phase: 5% water (0.1% $NH_4OH$) and 95% $CH_3CN$ from 90% water (0.1% $NH_4OH$) and 10% $CH_3CN$, 6.0 minutes, finally 0.5 minutes under these conditions] purity 96.6%, Rt = 3.365 minutes; MS Calcd.: 416; MS Found: 417 $([M+1]^+)$.

(Manufacturing example 3)

(Synthesis of K-26)

According to the following scheme, {4-[2-(4-fluoro-benzenesulfonyl-methyl-amino)-ethylsulfanil]-2,6-difluoro-phenoxy}-N,N-dimethyl-acetamide (K-26) was synthesized.

[Chem. 29]

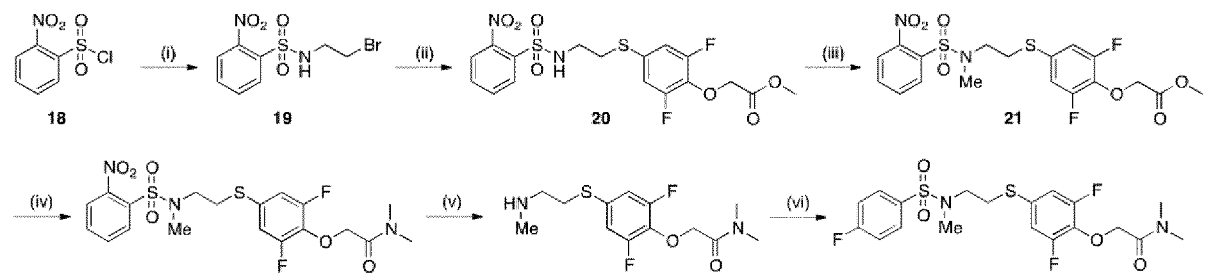


18 19 20 21 22 23 K-26

Step (i): Synthesis of N-(2-bromoethyl)-2-nitrobenzenesulfonamide (19)

2-nitrobenzenesulfonyl chloride (18) (5.0 g, 22.6 mmol) was dissolved in dehydrated dichloromethane (50 mL), and 2-bromoethylamine hydrobromide (5.55 g, 27.1 mmol) was added. Under an atmosphere of argon, the resultant solution was stirred at 0°C, and DIPEA (9.4 mL, 54.2 mmol) was gradually dropped. Thereafter, the solution was stirred at 0°C for 1 hour. 1 M HCl aq. was added, the resultant solution was moved to a separating funnel and the resultant product was extracted with EtAc three times. An organic layer was washed with brine and was dried with sodium sulfate. A desiccant was filtered, and the product was concentrated with an evaporator. The product was refined by silica gel column chromatography (hexane/EtOAc = 1:1), and thus 6.72 g (21.7 mmol, 96%) of a compound 19 was obtained as

a yellow solid.

Step (ii): Synthesis of methyl-2-(2,6-difluoro-4-((2-(2-nitrophenylsulfonamido)ethyl)thio)phenoxy)acetate (20)

To an acetone (7 mL) solution of the compound 4 (1.0 g, 4.27 mmol), $K_2CO_3$ (709 mg, 5.12 mmol) was added, and the resultant solution was stirred under an atmosphere of argon at room temperature for 20 minutes. The compound 19 (1.32 g, 4.27 mmol) was added, and the resultant solution was stirred under an atmosphere of argon at room temperature for 20 minutes. 1 M HCl aq. was added, the resultant solution was moved to a separating funnel and the resultant product was extracted with EtOAc three times. An organic layer was washed with brine and was dried with sodium sulfate. The product was filtered and was concentrated with an evaporator, and was then refined by silica gel column chromatography (hexane/EtOAc = 3:2), and thus 1.58 g (3.42 mmol, 80%) of a compound 20 was obtained as a yellow oily substance.

Step (iii): Synthesis of methyl-2-(2,6-difluoro-4-((2-(N-methyl-2- nitrophenylsulfonamido)ethyl)thio)phenoxy)acetate (21)

To a dehydrated DMF (4 mL) solution of the compound 20, $K_2CO_3$ (1.34 g, 9.72 mmol) and MeI (605 μL, 9.72 mmol) were added, and the resultant solution was stirred under an atmosphere of argon at room temperature for 1 hour. The solution was diluted with EtOAc, was then moved to a separating funnel and was washed with water. The resultant product was extracted with EtOAc three times, and was washed with brine. The product was dried with sodium sulfate, was filtered and was concentrated with an evaporator. The product was refined by silica gel column chromatography (hexane/EtOAc = 1:1), and thus 1.49 g (3.12 mmol, 96%) of a compound 21 was obtained as a yellow oily substance.

Step (iv): Synthesis of 2-(2,6-difluoro-4-((2-(N-methyl-2-nitrophenylsulfonamido)ethyl)thio)phenoxy)-N,N-dimethyl-acetamide (22)

A MeOH (2 mL) solution of dimethyl ammonium chloride (1.37 g, 16.8 mmol) was cooled with ice to 0°C under an atmosphere of argon, was subjected to addition of DIPEA (3.6 mL, 20.7 mmol) and was stirred at room temperature for 10 minutes. The compound 21 (400 mg, 0.840 mmol) was added, and the resultant solution was stirred at 70°C overnight. The reaction solution was cooled with ice, and 1 M HCL aq. was added. The solution was moved to a separating funnel, and the product was extracted with EtOAc three times, was then washed with brine and was dried with $Na_2SO_4$. The product was filtered and was then concentrated with an evaporator, and was refined by silica gel column chromatography (hexane/EtOAc = 1:1), and thus 390 mg (0.797 mmol, 95%) of a compound 22 was obtained as a yellow oily substance.

Step (v) : Synthesis of 2-(2,6-difluoro-4-((2-(methylamino)ethyl)thio)phenoxy)-N,N-dimethyl-acetamide (23)

The compound 22 (364 mg, 0.744 mmol) was dissolved in DMF (1.5 mL) and was cooled with ice to 0°C under an atmosphere of argon. 1-dodecanethiol (360 mL, 1.51 mmol) and $LiOH \cdot H_2O$ (63 mg, 1.51 mmol) were added, and the resultant solution was stirred under an atmosphere of argon at room temperature for 3 hours. The reaction solution was cooled to 0°C with ice, 1 M HCL aq. was then added until pH = 1 and the solution was washed with $CHCL_3$ three times. 1 M NaOH aq. was added until pH > 10, and the resultant product was then extracted with $CH_2Cl_2$ three times. The product was washed with brine and was dried with sodium sulfate. The product was filtered and was concentrated with an evaporator and a vacuum pump, and thus 172 mg (0.565 mmol, 76%) of a compound 23 was obtained as a colorless oily substance.

Step (vi): Synthesis of {4-[2-(4-fluoro-benzenesulfonyl-methyl-amino)-ethylsulfanil]-2,6-difluoro-phenoxy}-N,N-dimethyl-acetamide (K-26)

To a dehydrated $CH_2Cl_2$ (0.5 mL) solution of the compound 23 (35 mg, 0.115 mmol), DIPEA (24 μL, 0.138 mmol) was added. The resultant solution was cooled to 0°C with ice under an atmosphere of argon, and a dehydrated $CH_2Cl_2$ (0.5 mL) solution of 4-fluorobenzenesulfonyl chloride (24 mg, 0.121 mmol) was dropped. The temperature of the solution was returned to room temperature, and the solution was stirred for 10 minutes. 1 M HCL aq. was added while being cooled with ice, and the resultant product was extracted with EtOAc three times. The product was washed with brine and was dried with sodium sulfate. The product was filtered and was concentrated with an evaporator, and was then refined by PTLC (hexane/EtOAc = 1:6), and thus 47.5 mg (0.103 mmol, 90%) of a compound K-26 was obtained as a colorless oily substance.

(Manufacturing example 4)

(Synthesis of K-32 and K-30)

According to the following scheme, {4-[2-(benzenesulfonyl-methyl-amino)-ethylsulfanil]-2,6-difluoro-phenoxy}-N-methyl-acetamide (K-32) and {4-[2-(benzenesulfonyl-methyl-amino)-ethylsulfanil]-2,6-difluoro-phenoxy}-N,N-diethyl-acetamide (K-30) were synthesized.

[Chem. 30]

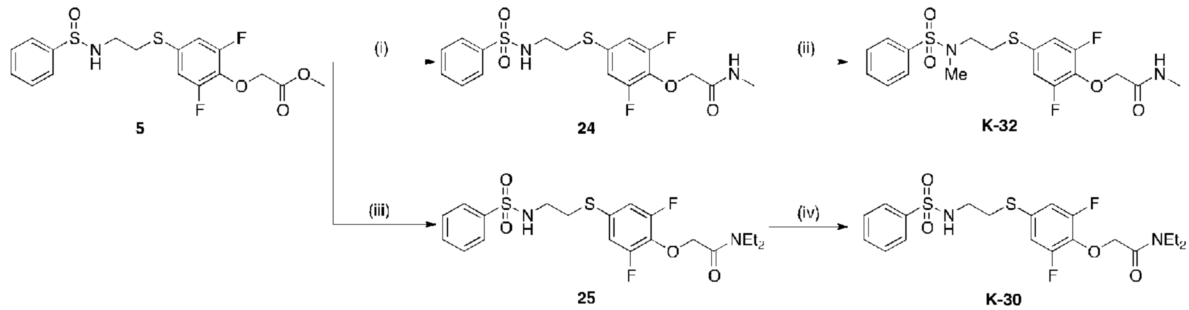


Step (i) : Synthesis of 2-(2,6-difluoro-4-((2-(phenylsulfonamido)ethyl)thio)phenyl)-N-methylacetamide (24)

To the compound 5 (1.19 g, 2.9 mmol), a methanol solution (9 mL) of methylamine was added, and the resultant solution was heated to reflux under an atmosphere of argon for 40 minutes. The solution was diluted with 1 M HCL, was then moved to a separating funnel and was washed with water. The resultant product was extracted with EtOAc three times, and was washed with brine. The product was dried with anhydrous sodium sulfate, was filtered and was concentrated with an evaporator. The product was refined by silica gel column chromatography (hexane/EtOAc = 2:3), and thus 1.12 g (2.7 mmol, 93%) of a compound 24 was obtained as a white solid.

Step (ii): Synthesis of {4-[2-(benzenesulfonyl-methyl-amino)-ethylsulfanil]-2,6-difluoro-phenoxy}-N-methyl-acetamide (K-32)

The compound 24 (0.51 g, 1.2 mmol) was dissolved in DMF (1.5 mL), $K_2CO_3$ (0.66 g, 4.8 mmol) was added, $CH_3I$ (0.3 mL, 4.8 mmol) was then added and the resultant solution was stirred under an atmosphere of argon at room temperature for 75 minutes. The solution was diluted with 1 M HCL, was then moved to a separating funnel and was washed with water. The resultant product was extracted with EtOAc three times, and was washed with brine. The product was dried with anhydrous sodium sulfate, was filtered and was concentrated with an evaporator. The product was refined by silica gel column chromatography (hexane/EtOAc = 2:3). Recrystallization was performed with a mixed solvent of hexane and ethyl acetate, and thus 0.38 g (0.88 mmol, 73%) of K-32 was obtained as while crystals.

Step (iii): Synthesis of 2-(2,6-difluoro-4-((2-(phenylsulfonamido)ethyl)thio)phenoxy)-N,N-diethylacetamide (25)

To a methanol solution (1 mL) of the compound 5 (0.51 g, 1.2 mmol), diethylamine (2.6 mL, 24.0 mmol) was added, and the resultant solution was heated to reflux under an atmosphere of argon for 12 hours. The solution was diluted with 1 M HCL, was then moved to a separating funnel and was washed with water. The resultant product was extracted with EtOAc three times, and was washed with brine. The product was dried with anhydrous sodium sulfate, was filtered and was concentrated with an evaporator. The product was refined by thin layer chromatography (hexane/EtOAc = 1:1), and thus 0.10 g (0.23 mmol, 19%) of a compound 25 was obtained as a yellow oily substance.

Step (iv): Synthesis of {4-[2-(benzenesulfonyl-methyl-amino)-ethylsulfanil]-2,6-difluoro-phenoxy}-N,N-diethyl-acetamide (K-30)

The compound 25 (0.10 g, 0.23 mmol) was dissolved in DMF (0.5 mL), $K_zCO_3$ (0.14 g, 0.92 mmol) was added, $CH_3I$ (0.06 mL, 0.92 mmol) was then added and the resultant solution was stirred under an atmosphere of argon at room temperature for 90 minutes. The solution was diluted with 1 M HCL, was then moved to a separating funnel and was

washed with water. The resultant product was extracted with EtOAc three times, and was washed with brine. The product was dried with anhydrous sodium sulfate, was filtered and was concentrated with an evaporator. The product was refined by silica gel column chromatography (hexane/EtOAc = 2:3), and thus 0.09 g (0.20 mmol, 86%) of K-30 was obtained as a yellow oily substance.

(Example 1)

(in vitro autoradiography method)

As rats, adult male SD rats (Charles River, Japan) which were 2 to 3 weeks old were used. An acute brain section which included the striatum and whose thickness was 200 μm was produced, was placed on a cell strainer and was left in an oxygenated ACSF (artificial cerebrospinal fluid) for 60 minutes. 12 ml of 100 μM PEPA (ACSF containing 1% DMSO) was adjusted, and [$^{11}$C] K-2 was added thereto so as to achieve 20 nM, and the resultant solution was left for 60 minutes. After the reaction, the resultant solution was washed with the ACSF for 10 seconds three times, and was finally washed with distilled water for 10 seconds. The cell strainer was cut out so as to be slightly larger than the brain section after the reaction, and was exposed to an imaging plate for 2 hours. The imaging plate after the exposure was imaged with Typhoon (GE Healthcare Japan) (resolution of 25 pixels).

(Electrophysiological verification)

Male SD rats which were 7 to 8 weeks old were used. The rat was decapitated under anesthesia with isoflurane, and an acute brain section which included the hippocampus and whose thickness was 400 μm was produced with a vibratome (VT1000; Leica Camera AG). The section was left in the ACSF at room temperature for 60 minutes, and then an AMPA current was measured by a whole-cell recording method. Under conditions in which the ACSF was refluxed at a rate of 3 ml/minute, only the AMPA current was isolated by administration of picrotoxin and APV. An exciting electrode was placed on a Schaffer fiber, and a recording electrode was placed on a pyramidal cell in CA1. The whole-cell recording was performed with a potential fixed to -80 mV, and a stimulus of 100 microseconds was applied every 30 seconds. The AMPA current in a ground state was recorded for 5 minutes, then K-2 or K-4 was administered for 15 minutes and thereafter the AMPA current was recorded for 30 minutes. When the section was left and when the section was refluxed, the ACSF was saturated with 95% $O_2$/5% $CO_2$ and was then used, and the composition of the ACSF was as follows; 119 mM NaCl, 2.5 mM KCl, 2.5 mM $CaCl_2$, 1.5 mM $MgSO_4$, 26 mM $NaHCO_3$ and 1 mM $NaH_2PO_4$. The recording electrode was produced such that the tip resistance thereof was 3 to 5 M ohms, and the composition of a filling liquid used was as follows; 115 mM $CsMeSO_4$, 20 mM CsCl, 10 mM HEPES, 2.5 mM $MgCl_2$, 4 mM $Na_2$ATP, 0.4 mM $Na_3$GTP, 10 Na-phosphocreatinine and 0.6 mM EGTA.

(Biological example)

(Preparation and administration of AMPA receptor-binding compound)

In a forced swimming test, AMPA receptor-binding compounds K-2 and K-4 were dissolved in 100% DMSO (Nacalai Tesque, Inc. Japan) so as to have a concentration of 2.1 mM, were diluted with saline immediately before being administered (15% DMSO, 320 μM) and were intravenously administered to the rat with an administered amount of 5 μl/weight (g) such that the administered amount fell within a range of 1 mg/kg to 1.5 mg/kg.

(Animal experiment)

In all animal experiments, the deliberation and the approval of the animal experiment committee in Yokohama City University were received (approval number: F-A-15-051). As rats, adult male Wistar rats which were 6 to 10 weeks old and Wistar Kyoto rats (WKY rats) which were depression model rats (Charles River, Japan) were used. In order to acquire a route for the repeated administration of K-2, a jugular vein cannula indwelling surgery was performed a week before the experiment. The rat was made to fall sleep with isoflurane (DS Pharma Animal Health, Japan), and thereafter while anesthesia was maintained at an isoflurane concentration of 1.5% (air 2 L/minute), the jugular vein cannula indwelling surgery was performed. The skin of the neck was incised about 3 cm such that subcutaneous fat was opened, and thus the jugular vein was exposed. A needle to which a cannula tube was fitted was made to penetrate the jugular vein, then the cannula tube was removed from the needle and the tip of the cannula tube was inserted 2.5 cm into the jugular vein so as to be fixed near the insertion portion with sutures. The opposite side of the cannula tube was exposed to the outside of the body through the back of the rat, a plug was fitted so as to prevent backflow and the opened skin was sutured. After the completion of the surgery, the rat was returned to a home cage.

(In vivo PET imaging using rats)

PET imaging was performed with microPET (Focus 220; Siemens Medical Solution). PET imaging experiment using the rat: After the rat was made to fall asleep in an anesthesia box filled with isoflurane (DS Pharma Animal Health, Japan), anesthesia was maintained at an isoflurane concentration of 1.5% (air 2 L/minute), and then an intravenous line was acquired from a tail vein with a 24G Surflo indwelling needle (Terumo Corporation, Japan). The rat was fixed to a PET shooting base, then radiation imaging for attenuation correction was performed before imaging and thereafter a radioactively labeled K-2 (about 40 MBq) was administered. During the PET imaging, the body temperature was maintained at 37 ± 0.5°C with a feedback type heating plate (BWT-100A; Bio Research Center, Japan). After the imaging, the intravenous line was removed, the administration of isoflurane was stopped, and then the rat was removed from the PET base and was returned to the home cage. For a period of one week after the imaging, the rat was raised in a room in which the imaging was performed, and then the rat was returned to a normal rat group raising room. A summation image was constructed, show-through was removed with a 0.5 mm Hanning filter and a dynamic image was reconstructed. The reconstructed image was analyzed with PMOD image analysis software (PMOD technologies) by integrating VOIs including a plurality of regions of the hippocampus, medial prefrontal cortex, nucleus accumbens, striatum, thalamus, cerebellum and the like with one formed on a template MRI image. The calculation value used in quantitative determination was %SUV (% of standardized uptake value) and was obtained by the following formula;

$$\%SUV = \text{amount of radiation of each tissue surrounded by VOI (kBq/cc)} / \text{administered amount of radiation (MBq)} \times \text{weight (kg)} \times 100$$

(Forced swimming test using rat)

The forced swimming test was performed in a pre-administration experiment on the day before the administration of the drug, in an acute administration experiment on the first day of the administration of the drug and in a chronic administration experiment on the seventh day of the administration of the drug. In the forced swimming test (the pre-administration experiment, the acute administration experiment and the chronic administration experiment), the rat was left for one hour so as to be habituated to the room where the experiment was performed. In the pre-administration experiment, immediately after the habituation, the forced swimming test was performed.

In the acute administration experiment, 15% DMSO containing saline or K-2 (administration concentration of 1.0 mg/kg, 0.1 mg/kg), K-4 (administration concentration of 1.0 mg/kg, 0.1 mg/kg), K-26 (administration concentration of 1.0 mg/kg), K-30 (administration concentration of 1.0 mg/kg) and K-32 (administration concentration of 1.0 mg/kg) which were dissolved in 15% DMSO containing saline were administered intravenously 30 minutes before the start of the forced swimming test.

In the chronic administration experiment, 15% DMSO containing saline, or K-2 (administration concentration of 1.0 mg/kg, 0.1 mg/kg) and K-4 (administration concentration of 1.0 mg/kg, 0.1 mg/kg) which were dissolved in 15% DMSO containing saline were administered intravenously for 7 days, and the forced swimming test was performed 30 minutes after the administration on the seventh day. The forced swimming test was performed with a cylindrical cage whose diameter was 20 cm and whose swimming height was 50 cm by injecting tap water having a temperature of 26±0.5°C up to a water level of 40 cm. The rat was put into the cylindrical cage, and the appearance of the rat swimming was shot for 10 minutes with a digital video camera (HC-V750, Panasonic, Japan). After the shooting, water droplets were wiped off the rat with a paper towel, and the rat was returned to the home cage.

In the analysis, in the acute administration experiment and the chronic administration experiment, a time during which the rat did not move the four limbs in minutes 2 to 10 of the shot video (minutes 0 to 2 were regarded as an environmental adaptation time) was measured as an immobility time. The immobility time was compared between the rat to which 15% DMSO containing saline was administered and the rats to which K-2, K-4, K-26, K-30 and K-32 dissolved in 15% DMSO containing saline were administered.

(PET imaging in humans)

PET imaging was performed on healthy men in their twenties. The man had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 120 minutes. For the imaging, a PET/CT device

"Aquiduo" (Toshiba Medical Systems Corporation) was used. In list data which was collected, dynamic images were reconstructed by an OS-EM method. The reconstructed image was analyzed with the PMOD image analysis software (PMOD technologies) by integrating VOIs including a plurality of regions of the frontal lobe, dentate cortex, hippocampus, amygdala, putamen, cerebellum, bridge and the like with one formed on a template MRI image. As the calculation value used in quantitative determination, %SUV (% of standardized uptake value) was used. As the %SUV of the three heathy persons, the average value for 70 minutes from 50 minutes after the administration of the PET drug to 120 minutes after the administration was calculated so as to be used for the analysis.

(Experiment result)

(Result of AMPA receptor binding test)

It was confirmed by the in vitro autoradiography method that K-2 indicated binding affinity to the AMPA receptors, and the Kd value thereof was 47.9 nM (Fig. 1). It was confirmed by electrophysiological verification that K-2 and K-4 indicated binding affinity to the AMPA receptors (Fig. 2).

(PET imaging on rat using AMPA receptor-labeled compound K-2)

A radioactively labeled K-2 ([$^{11}$C] K-2) was administered to the Wistar rat and the WKY rat, and PET imaging was performed in vivo (Figs. 3 and 4). Consequently, in the medial prefrontal cortex, striatum, cerebral cortex and thalamus of the WKY rat, a significantly lower uptake of the radioactively labeled K-2 in the brain was indicated (Fig. 5), and it was suggested that in these parts, the accumulated amount of AMPA receptor was lowered. It was found from the result thereof that in the WKY rat which was the depression model rat, the expression level of AMPA receptors was lowered in specific brain regions.

(Forced swimming test on rat using AMPA receptor-binding compound K-2 and K-2 similar compound)

In the acute administration experiment, it was found that there was no difference in the immobility time between the rat to which 15% DMSO containing saline was administered and the rat to which K-2 (administration concentration of 1.0 mg/kg, 1.5 mg/kg) was administered (Fig. 6).

In the rat to which K-4 (administration concentration of 1.0 mg/kg, 0.1 mg/kg) was administered, it was found that the immobility time was significantly lower, and the effect thereof tended to be greater in the case of 1.0 mg/kg than in the case of 0.1 mg/kg (Fig. 7). In the rats to which K-26, K-30 and K-32 were administered, there was a tendency that the immobility time was lower (Figs. 8, 9 and 10). As a result thereof, it was suggested that the acute administration of K-4 in the WKY rat which was the depression model rat indicated a volume-dependent antidepressant effect. It was also suggested that even in K-26, K-30 and K-32, an antidepressant effect was indicated.

In the chronic administration experiment, it was found that in the rats to which K-2 and K-4 were administered, the immobility time was significantly lower, and the effect thereof tended to be greater depending on its volume (Figs. 11 and 12). As a result thereof, it was suggested that the chronic administration of K-2 and K-4 in the WKY rat which was the depression model rat indicated an antidepressant effect.

Since the relationship was confirmed between the compounds such as K-2 an K-4 indicating binding affinity to the AMPA receptors and depression which was known to be related to the AMPA receptors, it is suggested that the antidepressant effects described above are caused by the activation of the AMPA receptor function by K-2, K-4, K-26, K-30 and K-32. Hence, it is suggested that K-2, K-4, K-26, K-30 and K-32 and compounds similar thereto in the present invention are useful for the therapy of not only depression but also diseases associated with the AMPA receptors.

(PET imaging on humans)

The uptake amounts were measured in the brains of the healthy persons to which [$^{11}$C] K-2 was administered. It is found from a graph shown in Fig. 13 that the ratio of the uptake amounts in parts within the brain is substantially uniform between a plurality of healthy persons (Y5, Y14 and Y16) and that individual differences are relatively low. In other words, it was confirmed that the PET imaging (Fig. 14) on the heathy persons to which [$^{11}$C] K-2 was administered was appropriate and that quantitative analysis in interpersonal comparison is highly reliable.

(Description of Abbreviations)

AMPA: α-amino-3-hydroxy-5-methyl-4-isoxazole-propionic acid
DIPEA: diisopropylethylamine
DCM: dichloromethane
EA, EtOAc: ethyl acetate
PE: petroleum ether
PEPA: 2-[2,6-difluoro-4-({2-[(phenylsulfonyl)amino]ethyl}thio)phenoxy] acetamide
PET: positron emission tomography
TEA: tetraethylammonium
TMS: tetramethylsilane
MeOH: methanol
EtOH: ethanol
DMF: N,N-dimethylformamide
MeI: methyl iodide
WSC·HCl: water-soluble carbodiimide hydrochloride
DMSO: dimethyl sulfoxide
ACSF: artificial cerebrospinal fluid

## Claims

1. A prophylactic and/or therapeutic agent for a disease associated with AMPA receptors, the prophylactic and/or therapeutic agent comprising a compound of formula (I) or a pharmaceutically acceptable salt or solvate thereof:

[Chem. 1]

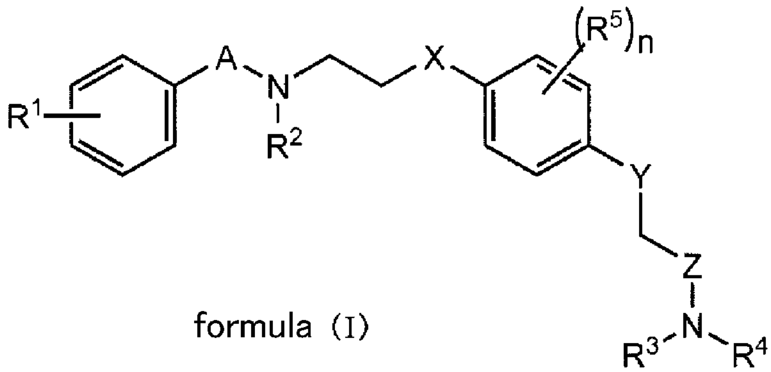

formula (I)

(in the formula,

each of A and Z independently represents CO, SO or $SO_2$;
each of X and Y independently represents S or O;
each of $R^1$ to $R^4$ independently represents hydrogen, alkyl, alkenyl, alkynyl or halo;
each $R^5$ independently represents alkyl, alkenyl, alkynyl or halo;
each $R^5$ independently represents alkyl, alkenyl, alkynyl or halo; and
n represents an integer of 0 to 4).

2. The prophylactic and/or therapeutic agent according to claim 1, wherein not all of $R^1$ to $R^4$ are hydrogen.

3. The prophylactic and/or therapeutic agent according to claim 1 or 2, wherein each of A and Z independently represents CO or $SO_2$.

4. The prophylactic and/or therapeutic agent according to any one of claims 1 to 3, wherein A represents $SO_2$ and Z represents CO.

5. The prophylactic and/or therapeutic agent according to any one of claims 1 to 4, wherein X represents S and Y represents O.

6. The prophylactic and/or therapeutic agent according to any one of claims 1 to 5, wherein $R^2$ represents alkyl, alkenyl or alkynyl.

7. The prophylactic and/or therapeutic agent according to claim 6, wherein $R^2$ represents alkyl.

8. The prophylactic and/or therapeutic agent according to any one of claims 1 to 7, wherein $R^1$ represents alkyl or halo.

9. The prophylactic and/or therapeutic agent according to any one of claims 1 to 8, wherein one of $R^3$ and $R^4$ represents hydrogen and the other represents alkyl.

10. The prophylactic and/or therapeutic agent according to any one of claims 1 to 9, wherein $R^5$ represents halo.

11. The prophylactic and/or therapeutic agent according to claim 10, wherein the halo is fluoro.

12. The prophylactic and/or therapeutic agent according to any one of claims 1 to 11, wherein n represents 2.

13. The prophylactic and/or therapeutic agent according to claim 1, wherein the prophylactic and/or therapeutic agent is selected from the group consisting of compounds below.

[Chem. 2]

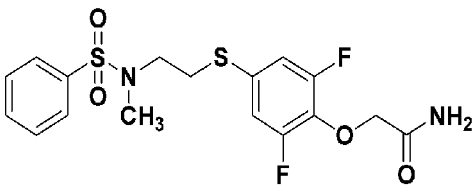
;
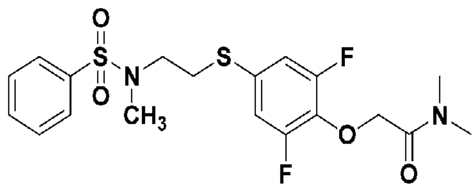

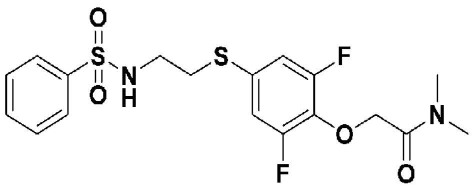
;
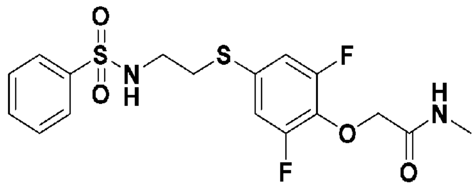

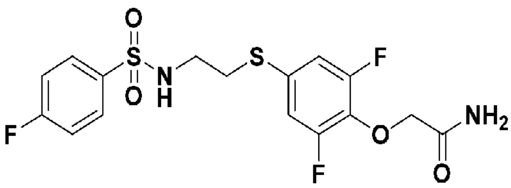
;
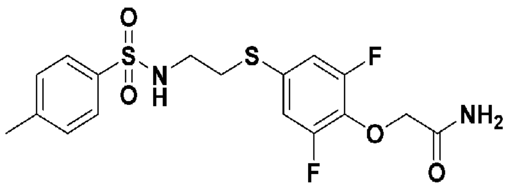

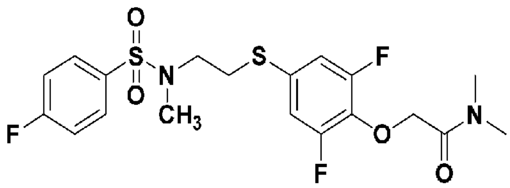
;
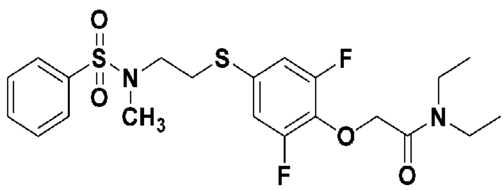

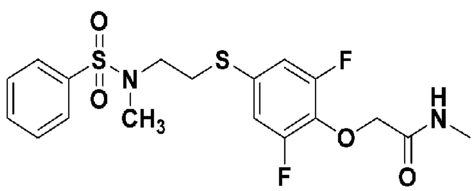

**14.** The prophylactic and/or therapeutic agent according to claims 1 to 13, wherein the prophylactic and/or therapeutic agent is an AMPA receptor function activator.

**15.** The prophylactic and/or therapeutic agent according to any one of claims 1 to 13, wherein the disease associated with the AMPA receptors is a mental disease or a neurological disease.

**16.** The prophylactic and/or therapeutic agent according to claim 15, wherein the mental disease is depression.

FIG. 1

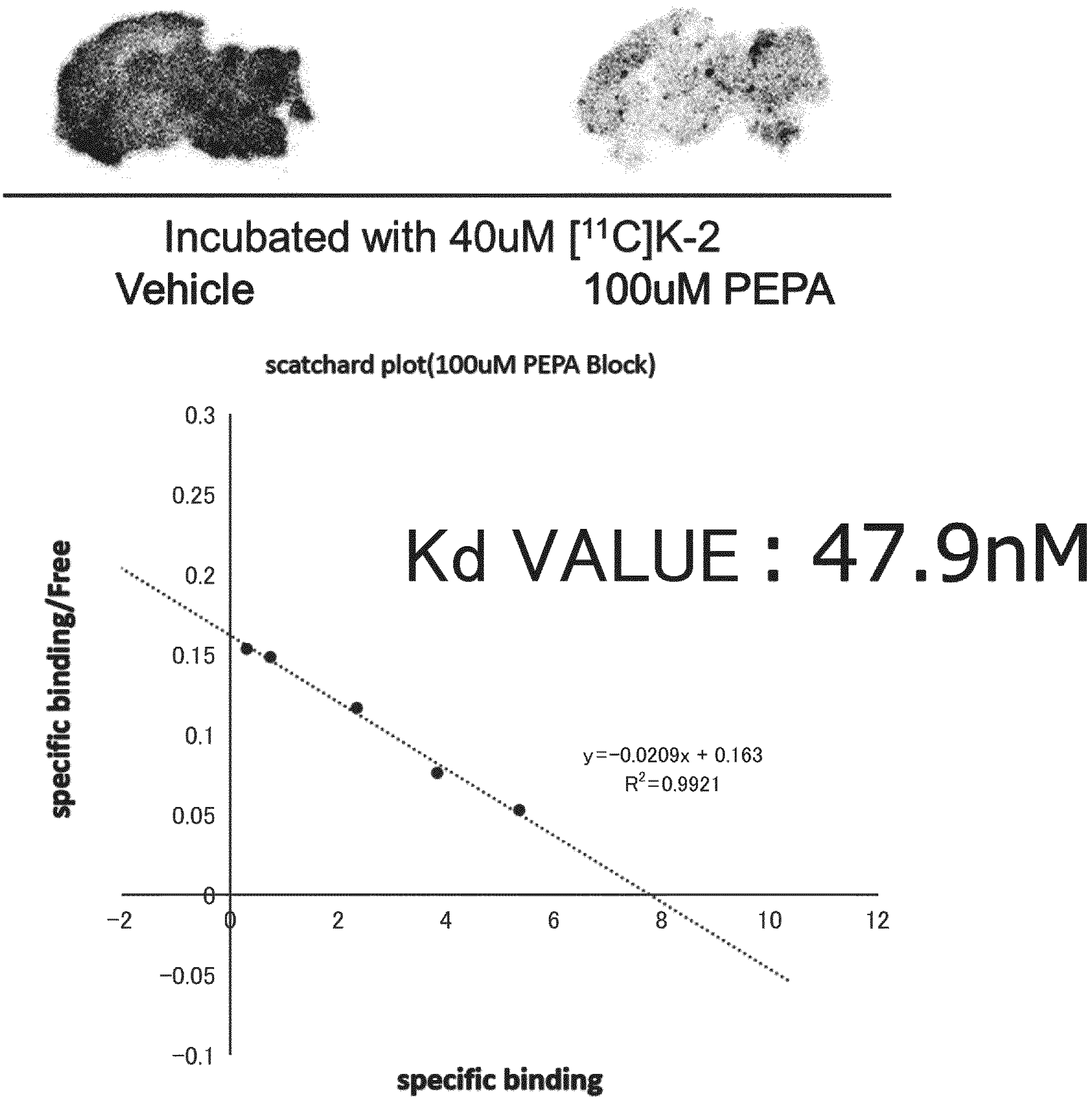
Incubated with 40uM [11C]K-2
Vehicle
100uM PEPA
scatchard plot(100uM PEPA Block)
Kd VALUE : 47.9nM
y=−0.0209x + 0.163
R2=0.9921
specific binding/Free
specific binding
0.3
0.25
0.2
0.15
0.1
0.05
0
−0.05
−0.1
−2
0
2
4
6
8
10
12

FIG. 2

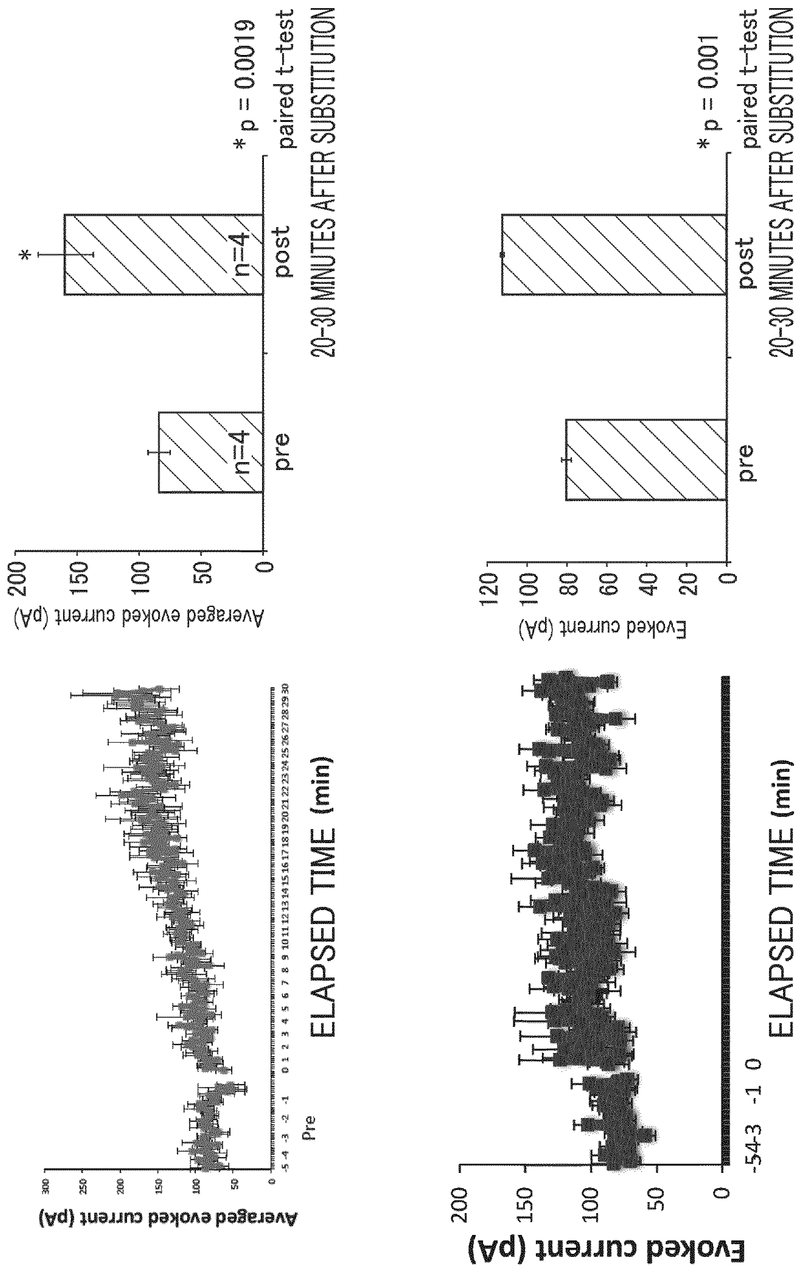
Averaged evoked current (pA)
ELAPSED TIME (min)
Pre
Averaged evoked current (pA)
pre
post
n=4
n=4
*
* p = 0.0019
paired t-test
20-30 MINUTES AFTER SUBSTITUTION
Evoked current (pA)
ELAPSED TIME (min)
-54-3 -1 0
Evoked current (pA)
pre
post
* p = 0.001
paired t-test
20-30 MINUTES AFTER SUBSTITUTION

K-2

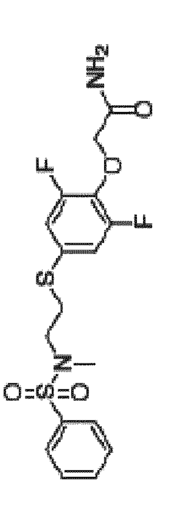

K-4

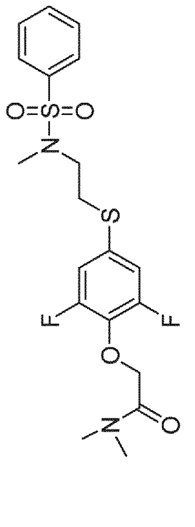

FIG. 3

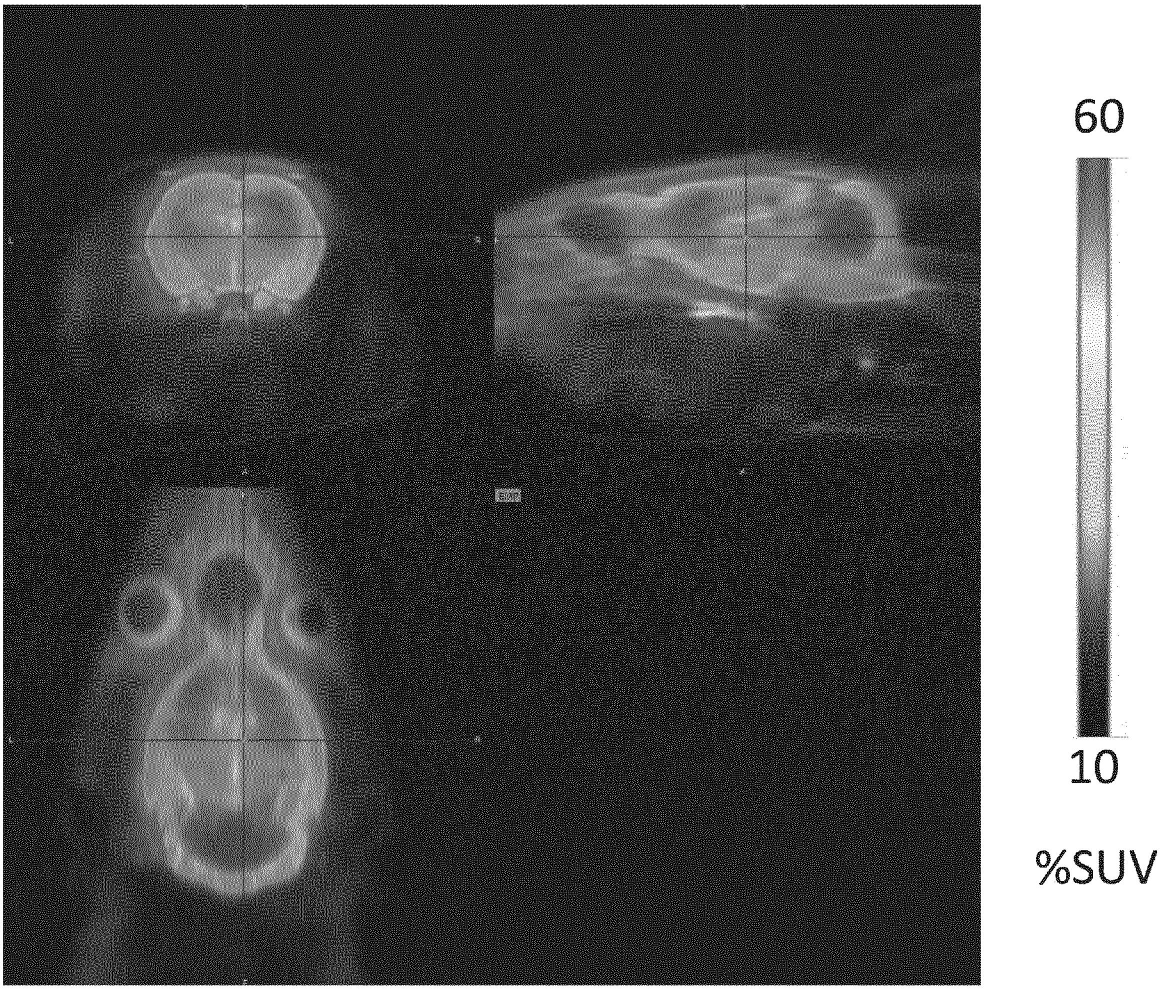

FIG. 4

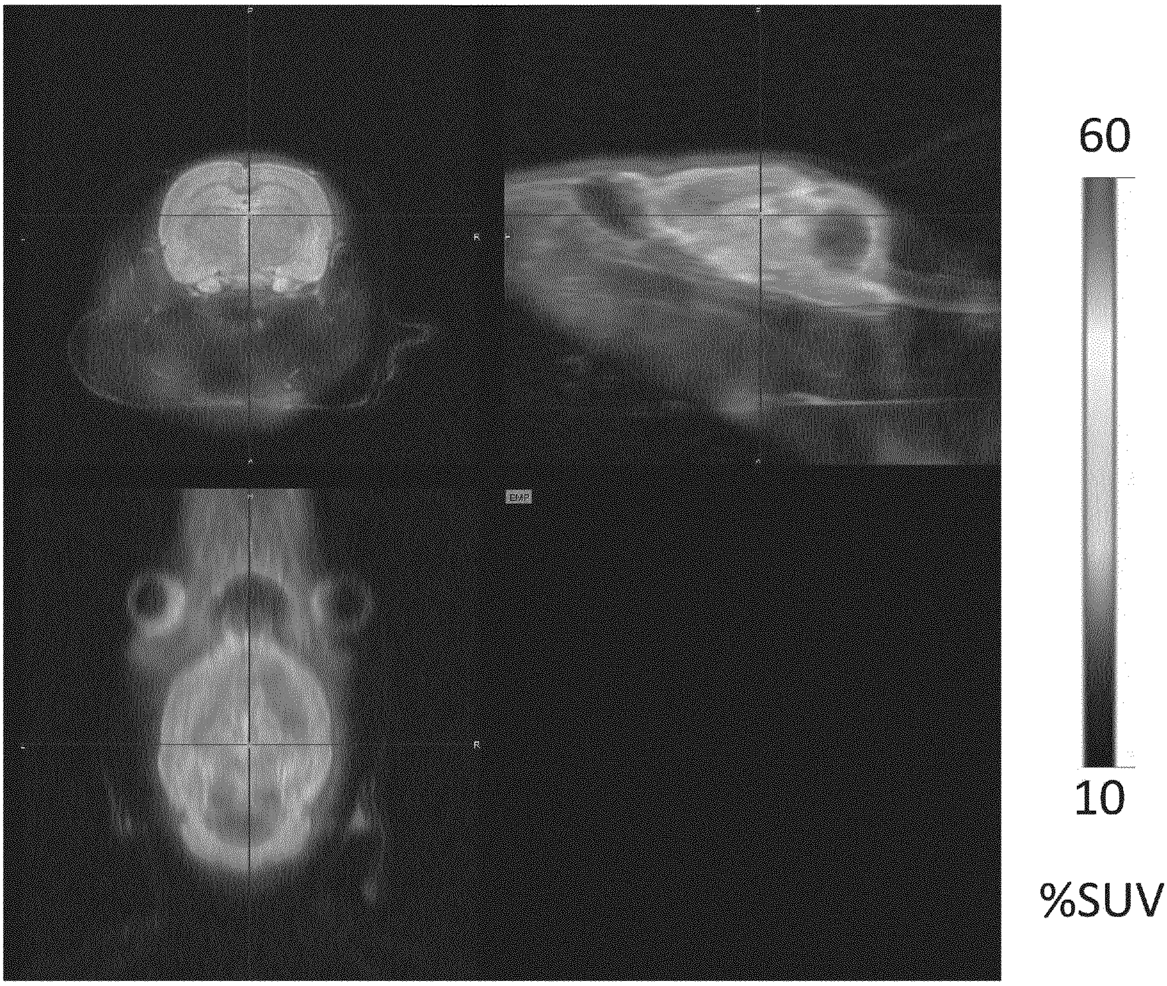

FIG. 5

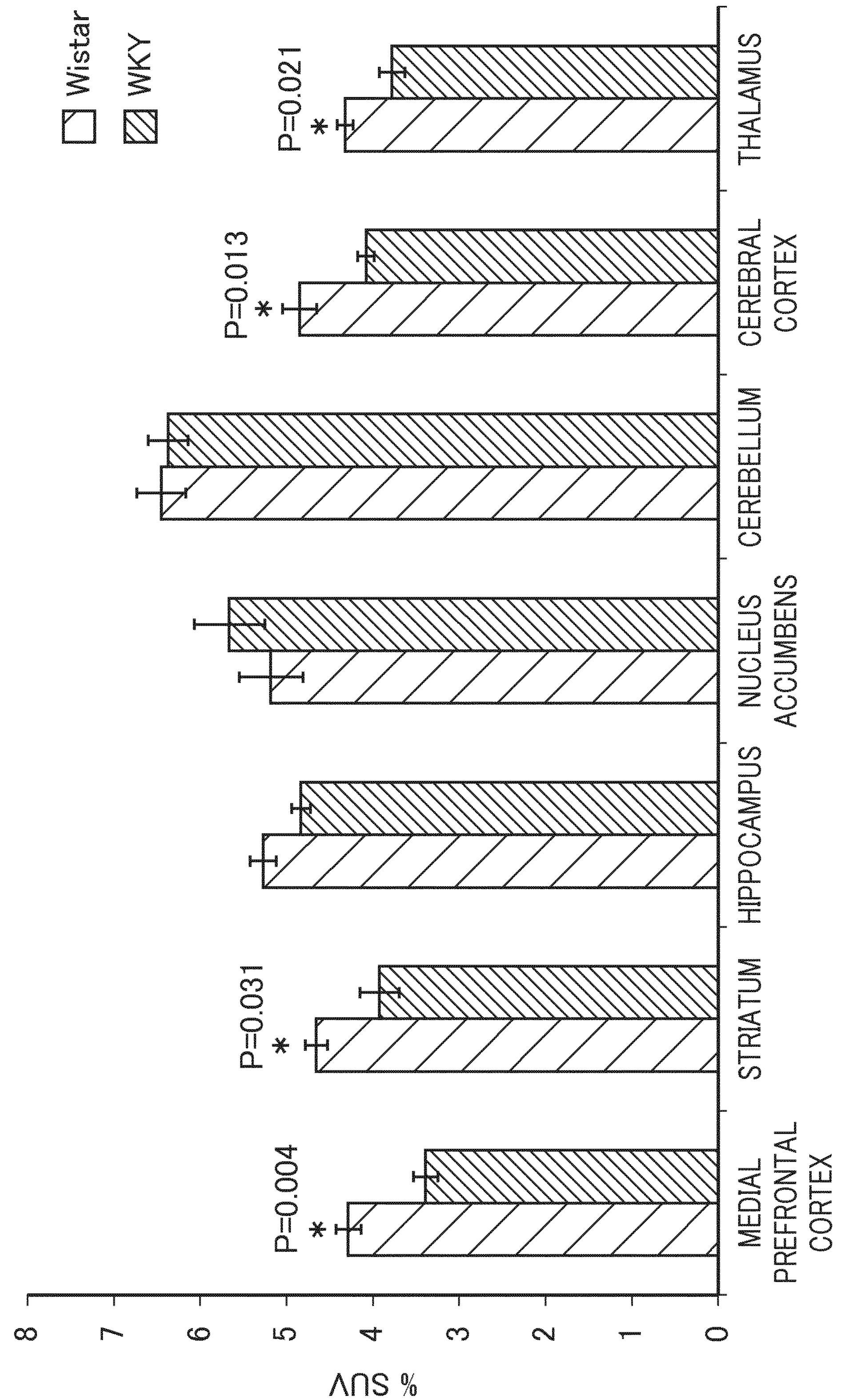
Wistar
WKY
% SUV
0
1
2
3
4
5
6
7
8
MEDIAL PREFRONTAL CORTEX
STRIATUM
HIPPOCAMPUS
NUCLEUS ACCUMBENS
CEREBELLUM
CEREBRAL CORTEX
THALAMUS
P=0.004
*
P=0.031
*
P=0.013
*
P=0.021
*

FIG. 6

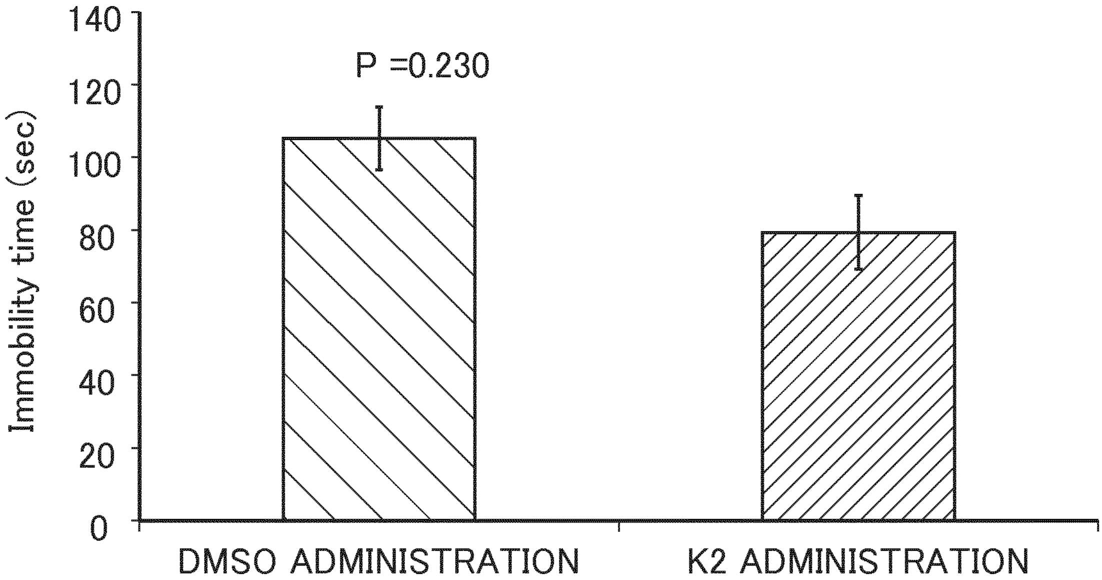
140
120
100
80
60
40
20
0
Immobility time (sec)
P =0.230
DMSO ADMINISTRATION
K2 ADMINISTRATION

FIG. 7

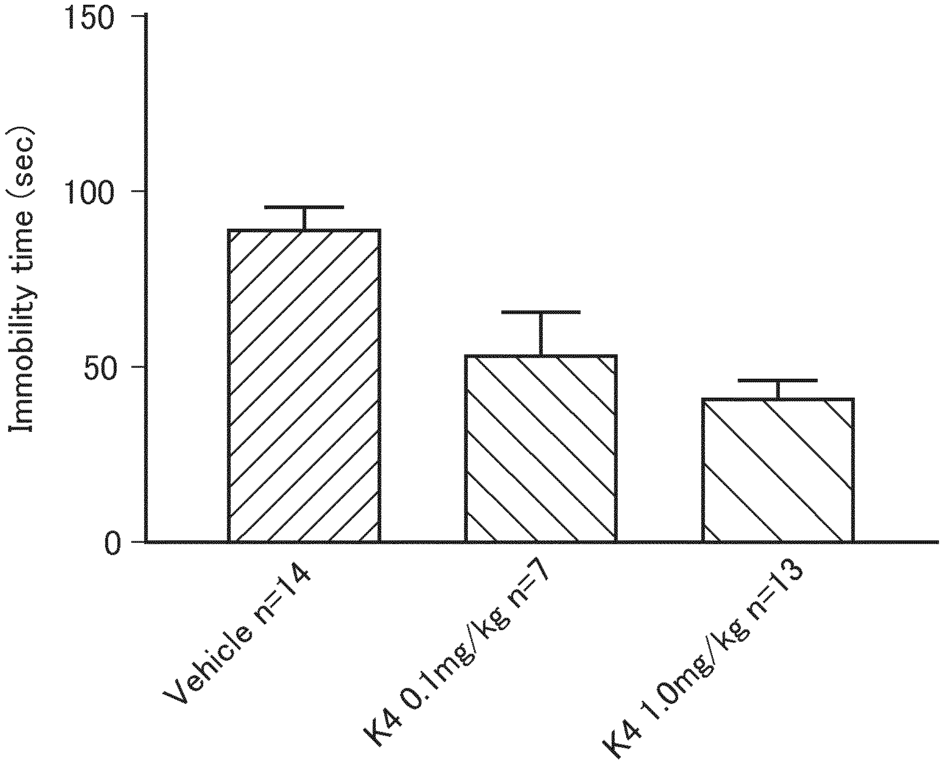
150
100
50
0
Immobility time (sec)
Vehicle n=14
K4 0.1mg/kg n=7
K4 1.0mg/kg n=13

FIG. 8

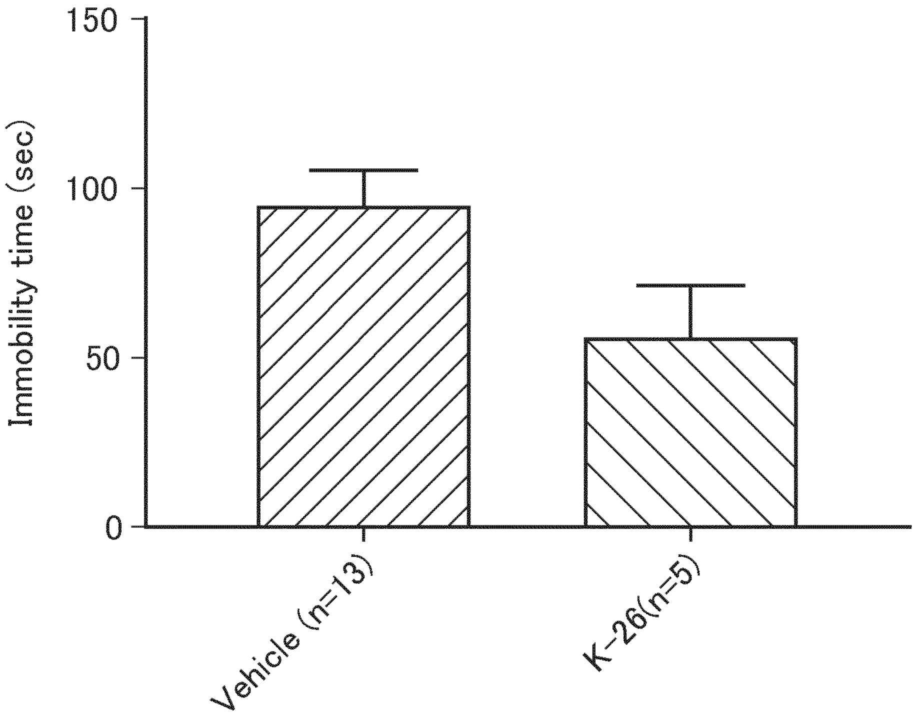
150
100
50
0
Immobility time (sec)
Vehicle (n=13)
K-26(n=5)

FIG. 9

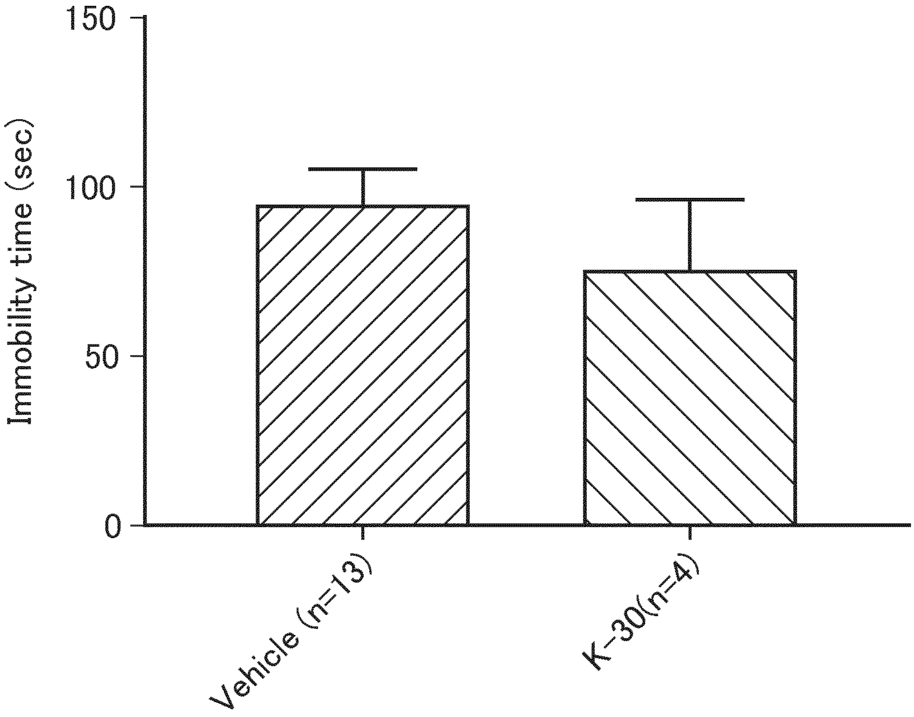
150
100
50
0
Immobility time (sec)
Vehicle (n=13)
K-30(n=4)

FIG. 10

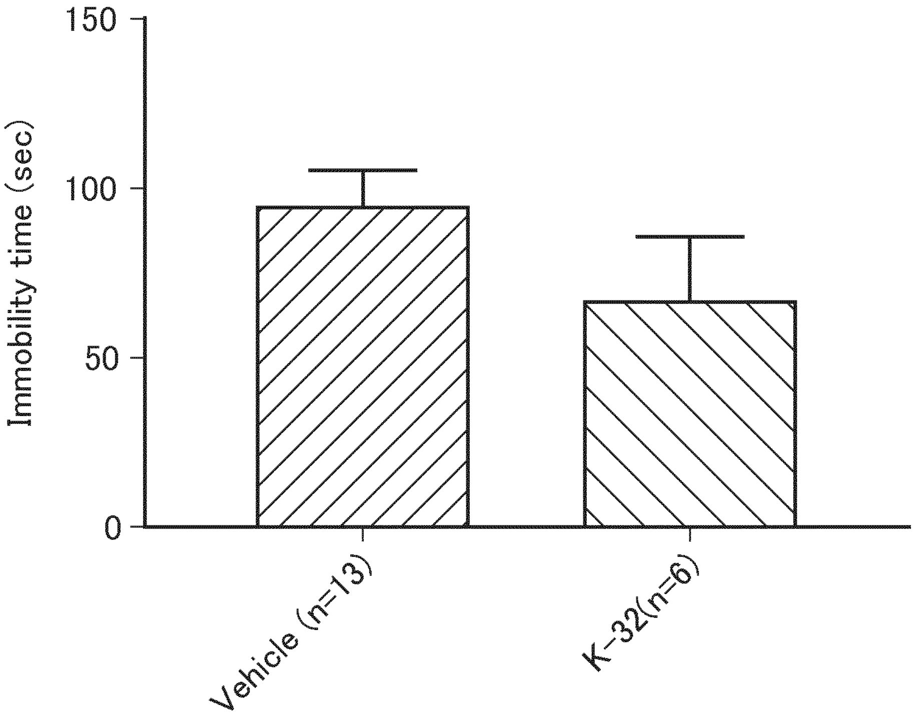
150
100
50
0
Immobility time (sec)
Vehicle (n=13)
K-32(n=6)

FIG. 11

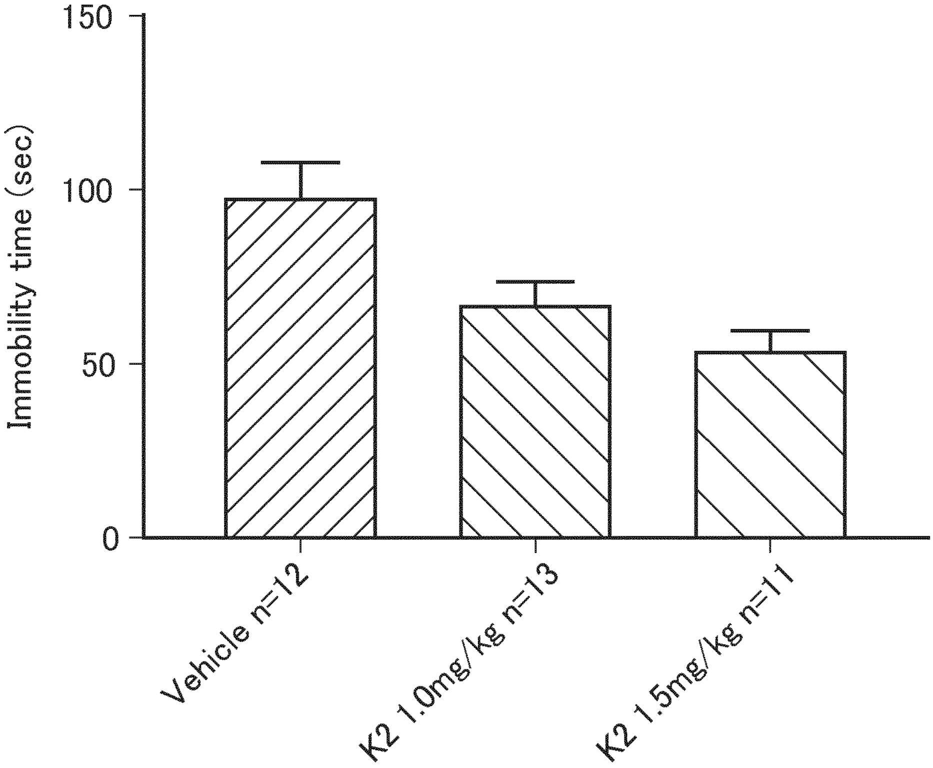
150
100
50
0
Immobility time (sec)
Vehicle n=12
K2 1.0mg/kg n=13
K2 1.5mg/kg n=11

FIG. 12

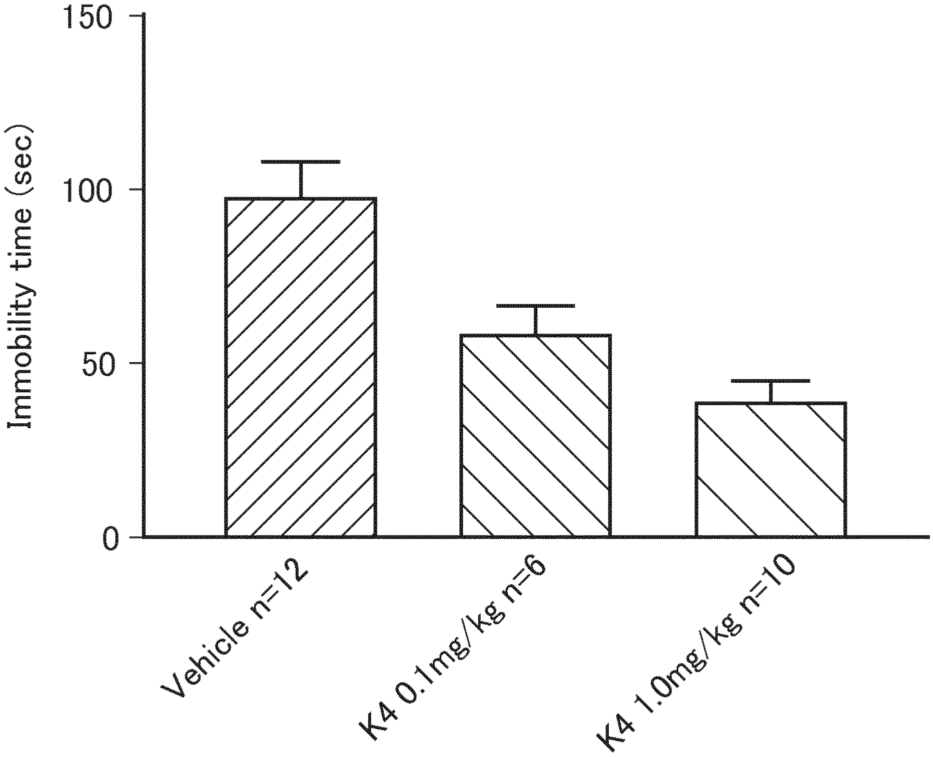
150
100
50
0
Immobility time (sec)
Vehicle n=12
K4 0.1mg/kg n=6
K4 1.0mg/kg n=10

FIG. 13

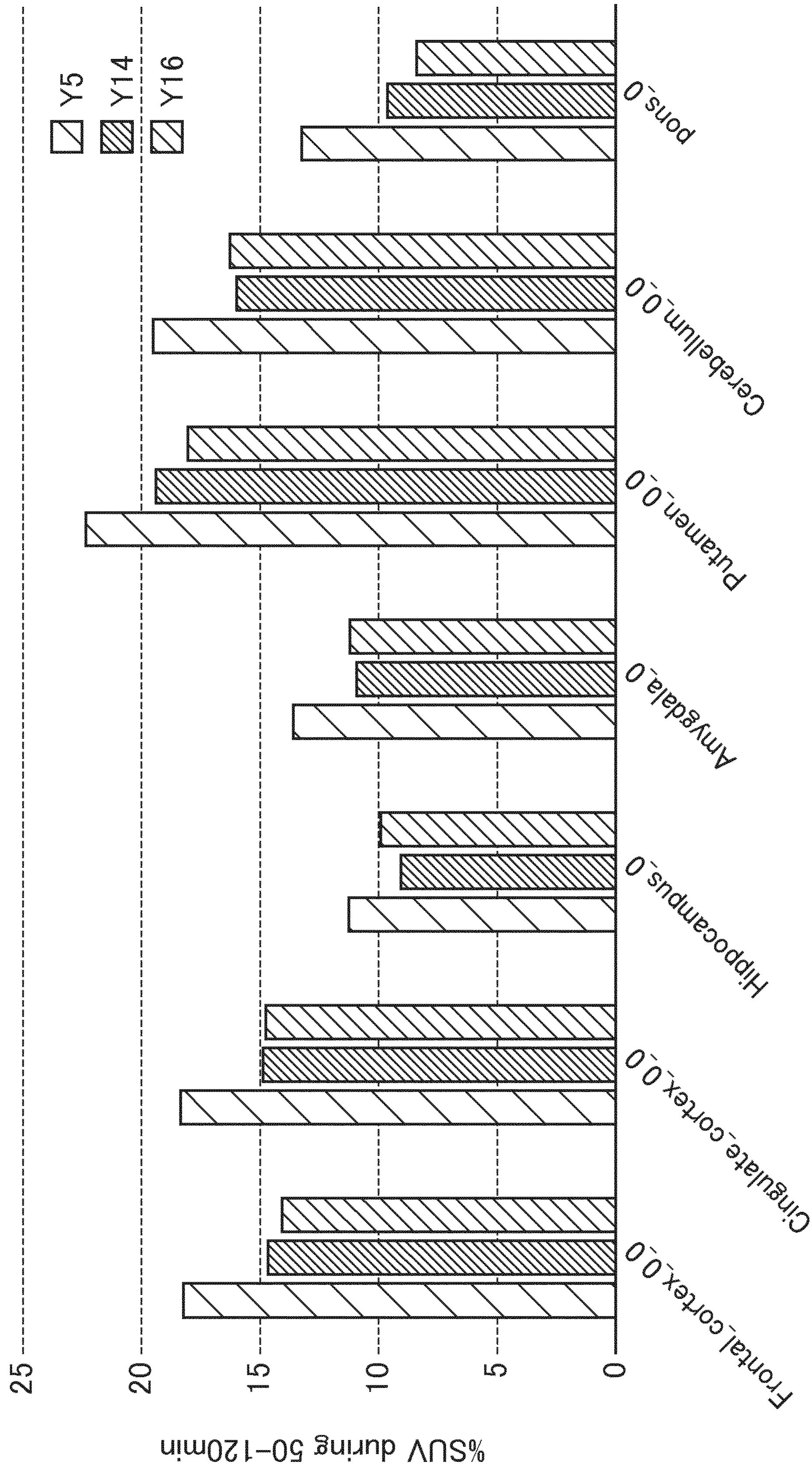

Y5
Y14
Y16
%SUV during 50−120min
0
5
10
15
20
25
Frontal_cortex_0_0
Cingulate_cortex_0_0
Hippocampus_0
Amygdala_0
Putamen_0_0
Cerebellum_0_0
pons_0

FIG. 14

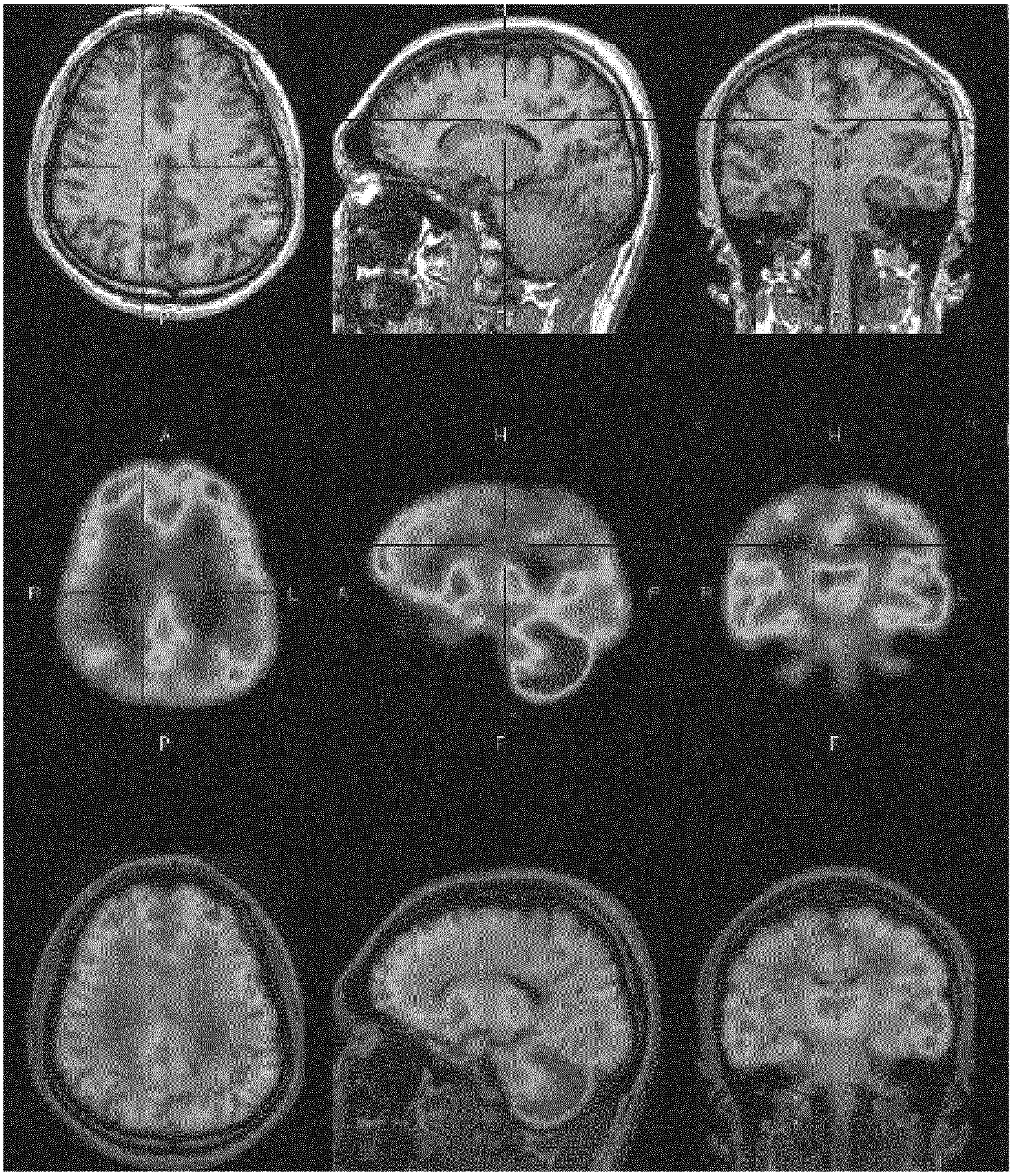

## INTERNATIONAL SEARCH REPORT

International application No.
PCT/JP2018/000531

**A. CLASSIFICATION OF SUBJECT MATTER**

Int.Cl. A61K31/18(2006.01)i, A61P25/00(2006.01)i, A61P25/24(2006.01)i, A61P43/00(2006.01)i

According to International Patent Classification (IPC) or to both national classification and IPC

**B. FIELDS SEARCHED**

Minimum documentation searched (classification system followed by classification symbols)
Int.Cl. A61K31/18, A61P25/00, A61P25/24, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | US 2015/0125441 A1 (NEW YORK UNIVERSITY) 07 May 2015, claims 1-6, example 6 & WO 2013/177484 A1 | 1, 3-5, 10-12, 14-16<br>1-5, 8-16<br>6, 7 |
| X<br>Y<br>A | YAMADA, D. et al., "Facilitating actions of an AMPA receptor potentiator upon extinction of contextually conditioned fear response in stressed mice", Neuroscience Letters, 2011, vol. 488, pp. 242-246, ISSN 0304-3940, in particular, abstract, fig. 2 | 1, 3-5, 10-12, 14, 15<br>1-5, 8-16<br>6, 7 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 March 2018 (13.03.2018) | 27 March 2018 (27.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/000531

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 1996/25926 A1 (NIPPON SUISAN KAISHA, LTD.) 29 August 1996, claims, examples & US 5955505 A, claims 1-3, example 16 & EP 811375 A1 | 1-5, 8-16<br>6, 7 |
| P, X | WO 2017/006931 A1 (YOKOHAMA CITY UNIVERSITY) 12 January 2017, claims 1-12, paragraph [0039], example 6 & TW 201706242 A | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TAKAHASHI.** *Science,* 2003
- **MITSUSHIMA ; TAKAHASHI.** *PNAS,* 2011
- **JITSUKI ; TAKAHASHI.** *Neuron,* 2011
- **MIYAZAKI ; TAKAHASHI.** *J. Clinical Investigation,* 2012
- **MIYAZAKI ; TAKAHASHI.** *European J. Neurosci.,* 2013
- **MITSUSHIMA ; TAKAHASHI.** *Nature Communications,* 2013
- **TADA ; MIYAZAKI ; TAKAHASHI.** *PNAS,* 2016
- **TAKEMOTO ; TAKAHASHI.** *Nat.Biotech.,* 2016
- **JINGLI ZHANG et al.** *Rev. Neurosci.,* 2013, vol. 24 (5), 499-505
- **SIMON E WARD et al.** *British Journal of Pharmacology,* 2010, vol. 160, 181-190